# EUROPEAN PATENT APPLICATION

(11) **EP 4 498 078 A1**
(43) Date of publication of application: **29.01.2025**
(21) Application number: 23774254.9
(22) Date of filing: 02.02.2023
(51) Int. Cl.: G01N 30/46, B01D 15/00, C07K 1/16, C07K 16/00, C12M 1/00

(54) **SIMULATED MOVING-BED CHROMATOGRAPHIC SEPARATION METHOD AND SIMULATED MOVING-BED CHROMATOGRAPHIC SEPARATION SYSTEM**

(30) Priority: 22.03.2022 JP 2022045536
(71) Applicant: Organo Corporation, Tokyo 136-8631 (JP)
(72) Inventor: OGINO, Masahiro, Tokyo 136-8631 (JP); OKADA, Kazuo, Tokyo 136-8631 (JP); SATO, Kohei, Tokyo 136-8631 (JP); TSURUTA, Masaki, Tokyo 136-8631 (JP)
(74) Representative: Müller-Boré & Partner Patentanwälte PartG mbB
(86) International application number: PCT/JP2023/003433
(87) International publication number: WO 2023/181655

(57) **Abstract**

A simulated moving-bed type chromatographic separation method including: using a circulation system in which three or more unit packed columns each filled with an adsorbent are connected in series and in an endless form via pipes, dividing the circulation system into at least three sections annularly continuous from an upstream side to a downstream side so that each section has at least one of the unit packed column, and separating, by using an eluent, a weakly adsorptive component, a strongly adsorptive component, and an intermediately adsorptive component having adsorption performance on the adsorbent, which performance is intermediate between the previous two components, the weakly adsorptive component, strongly adsorptive component, and intermediately adsorptive component being included in a feed solution, wherein a position of supplying the feed solution, a position of extracting the weakly adsorptive fraction, a position of extracting the intermediately adsorptive fraction, and a position of extracting the strongly adsorptive fraction have a specific relationship; and
a chromatographic separation system suitable for carrying out the chromatographic separation method.

## Description

### FIELD OF THE INVENTION

The present invention relates to a simulated moving-bed type chromatographic separation method and a simulated moving-bed type chromatographic separation system.

### BACKGROUND OF THE INVENTION

In chromatographic separation by a simulated moving-bed scheme, a circulation system is constructed in which a plurality of unit packed columns (hereinafter, also simply referred to as "packed columns", and may be called "columns") packed with an adsorbent having a selective adsorption ability with respect to a specific component of two or more components contained in a feed solution are connected in series via pipes and the packed column at the most downstream portion and the packed column at the most upstream portion are connected in an endless form. A feed solution and an eluent are supplied to this circulation system, a fraction with a fast moving velocity (weakly adsorptive fraction), a fraction with a slow moving velocity (strongly adsorptive fraction), and, if necessary, a fraction with an intermediate moving velocity (intermediately adsorptive fraction) in the circulation system are extracted from different positions, respectively, and then, the feed solution supply position, the eluent supply position, the extraction position of the weakly adsorptive fraction, the extraction position of the intermediately adsorptive fraction, and the extraction position of the strongly adsorptive fraction are shifted toward the fluid circulation direction of the circulation system while maintaining a certain positional relationship. By repeating this operation, processing operations of the moving bed capable of continuously performing feed solution supply are artificially achieved.

For example, Patent Literature 1 describes a separation method having a multicomponent system including an endless circulation flow path formed in series using a plurality of unit packed layers filled with an adsorbent so that the circulation flow path is provided so as to enable circulation and blocking, the method comprising: a first step of flowing a feed fluid containing three or more components with different affinities to the adsorbent through the plurality of unit packed layers to supply, while blocking circulation of the system at a position upstream of an adsorption band formed using a preselected component among low-affinity components, the feed fluid to the system at a position downstream of the blocked portion, the system including adsorption bands divided, in sequence, from a component with lowest affinity for the adsorbent to a component with strongest affinity, and extracting, from the system, a fraction enriched with a predetermined component among the components forming each adsorption band at a position upstream of the blocked position; and a second step of extracting, while circulating the system without supplying the feed fluid, each fraction enriched with each component divided into adsorption bands from which the component is not extracted in the first step by supplying a desorbent fluid from a position upstream of the adsorption band, and sequentially shifting each position, where each fraction above is extracted, toward the downstream side of the circulation flow, wherein the above steps are repeated as one cycle.

The chromatographic separation of the simulated moving-bed scheme has been also studied on application to medical fields since a target substance to be purified can be continuously obtained at a high purity. For example, in production of antibody drugs, in an extract or culture medium of cultured cells producing antibodies, in addition to target antibodies, fragments, which have been generated by cleavage or the like of antibodies and do not function as antibodies, or aggregates, which are generated by aggregation of antibodies to become bulky, are generated. In general, the fragments have a few interaction sites with the adsorbent and weak adsorptive property with respect to the adsorbent. On the other hand, the aggregates have strong adsorptive property with respect to the adsorbent. Therefore, in the case of applying the chromatographic separation of the simulated moving-bed scheme to purification of antibody drugs, it is necessary to fractionate a target antibody as an intermediately adsorptive fraction showing intermediate adsorptive property with respect to the adsorbent. On the other hand, regarding a weakly adsorptive fraction and a strongly adsorptive fraction, it is necessary to sufficiently remove both of the fractions with a high removal rate.

For example, Patent Literature 2 describes a simulated moving-bed type chromatographic separation method including using a circulation system in which a plurality of unit packed columns each packed with an adsorbent are connected in series and in an endless form via pipes, and separating, by using two or more kinds of eluents, a weakly adsorptive component, a strongly adsorptive component, and an intermediately adsorptive component that has adsorptive property intermediate between the previous two components, with respect to the adsorbent, the weakly adsorptive component, the strongly adsorptive component, and the intermediately adsorptive component being contained in a feed solution, wherein a feed solution supply port F, two or more eluent supply ports D corresponding to the two or more kinds of eluents, an extraction port A of a weakly adsorptive fraction containing the weakly adsorptive component, an extraction port B of an intermediately adsorptive fraction containing the intermediately adsorptive component, and an extraction port C of a strongly adsorptive fraction containing the strongly adsorptive component are provided on the pipes of the circulation system, and positions of the feed solution supply port F, the extraction port A, the extraction port B, and the extraction port C are set to have a specific positional relationship, and thus describes that the purification target component in the feed solution can be collected in high purity while using a smaller amount of adsorbent used.

### CITATION LIST

### PATENT LITERATURES

Patent Literature 1: JP-B-H7-24724 ("JP-B" means an examined published Japanese patent)
Patent Literature 2: JP-A-2020-085881 ("JP-A" means an unexamined published Japanese patent application)

### SUMMARY OF THE INVENTION

### TECHNICAL PROBLEM

To date, some technologies have been proposed that improve the purification efficiency of a target component by devising the supply position and the supply timing of a feed solution or an eluent and the extraction position and the extraction timing of each fraction in a simulated moving-bed type chromatographic separation. On the other hand, there are a wide variety of components to be separated, and there are also a wide variety of contaminants in a sample containing the components. Thus, a simulated moving-bed type chromatographic separation technology effective for separation and purification of a certain component in a feed solution sample may be applied to separation and purification of a specific component in another feed solution sample. This case may result in the situation where the specific component cannot be sufficiently separated in high purity and high efficiency. Therefore, in order to broaden the application range of the simulated moving-bed type chromatographic separation, it is desirable to increase variations of technologies capable of purifying a specific component with high purity.

In addition, in the conventional simulated moving-bed type chromatographic separation method as described in Patent Literature 2, the feed solution supply port, each eluent supply port, and each fraction extraction port are simultaneously shifted to the downstream side after the end of one cycle of operation. However, it has also been found that in this method, the purification target substance may be insufficiently separated depending on the purification target substance of interest.

Here, the present invention provides a chromatographic separation method that makes it possible to fractionate a purification target component in a feed solution with high purity while setting the supply position and the supply timing of the feed solution and/or an eluent, and the extraction position and the extraction timing of each fraction to be different from conventional methods.

Furthermore, the present invention also provides a chromatographic separation system suitable for implementing the above-described chromatographic separation method.

### SOLUTION TO PROBLEM

The above problems of the present invention have been solved by the following means.
[1] A simulated moving-bed type chromatographic separation method including: using a circulation system in which three or more unit packed columns each filled with an adsorbent are connected in series and in an endless form via pipes, dividing the circulation system into at least three sections annularly continuous from an upstream side to a downstream side so that each section has at least one of the unit packed column, and separating, by using an eluent, a weakly adsorptive component, a strongly adsorptive component, and an intermediately adsorptive component having adsorption performance on the adsorbent, which performance is intermediate between the previous two components, the weakly adsorptive component, strongly adsorptive component, and intermediately adsorptive component being included in a feed solution, wherein a cycle containing a first process and a second process below is repeated:
   <first process>
      a process containing blocking circulation of the circulation system by setting a blocked position to a position upstream of the weakly adsorptive component-rich section, supplying the feed solution to a section downstream of the blocked position, and extracting, from the circulation system, the intermediately adsorptive component-rich intermediately adsorptive fraction from a section upstream of the blocked position; and
   <second process>
      a process containing extracting the strongly adsorptive component-rich strongly adsorptive fraction by controlling supply of the eluent and blocking of the circulation system without supplying the feed solution, and shifting a supply position of the eluent relative to the circulation system and an extraction position of the strongly adsorptive fraction to a downstream side in accordance with movement of the strongly adsorptive fraction, and
   wherein, in the cycle containing the first process and the second process, the extraction position of the strongly adsorptive fraction is shifted to a downstream side by 2 sections or more and the [number of sections] shifted toward the downstream side and the total number of sections are not the same.
[2] A simulated moving-bed type chromatographic separation method including: using a circulation system in which three or more unit packed columns each filled with an adsorbent are connected in series and in an endless form via pipes, dividing the circulation system into at least three sections annularly continuous from an upstream side to a downstream side so that each section has at least one of the unit packed column, and separating, by using an eluent, a weakly adsorptive component, a strongly adsorptive component, and an intermediately adsorptive component having adsorption performance on the adsorbent, which performance is intermediate between the previous two components, the weakly adsorptive component, strongly adsorptive component, and intermediately adsorptive component being included in a feed solution, wherein a cycle containing step (A) and step (B) below is repeated:
   <step (A)>
      a step including:
      sub-step (A1) of blocking circulation of the circulation system by setting a blocked position to a position upstream of the weakly adsorptive component-rich section, supplying the feed solution at a section downstream of the blocked position and extracting, from the circulation system, the weakly adsorptive component-rich weakly adsorptive fraction at a position further downstream of the blocked position, and supplying an eluent for desorbing the intermediately adsorptive component at a section upstream of the blocked position and extracting, from the circulation system, the intermediately adsorptive component-rich intermediately adsorptive fraction at a position downstream thereof,
      sub-step (A2) of supplying an eluent for desorbing the weakly adsorptive component instead of supplying the feed solution and continuing extraction of the weakly adsorptive fraction and extraction of the intermediately adsorptive fraction,
      sub-step (A3) of shifting the blocked position to an upstream side and supplying an eluent for desorbing the strongly adsorptive component at a position upstream of the blocked position having been shifted and extracting the strongly adsorptive component-rich strongly adsorptive fraction at a position further downstream thereof while continuing extraction of the weakly adsorptive fraction, and
      sub-step (A4) of shifting the extraction position of the weakly adsorptive fraction to a downstream side and extracting the weakly adsorptive fraction while extracting neither the intermediately adsorptive fraction nor the strongly adsorptive fraction,
      wherein, in step (A), the sub-steps (A1), (A2), (A3) and (A4) are performed in this sequence; and
   <step (B)>
      a step including step α of separately extracting each of the weakly adsorptive fraction or strongly adsorptive fraction not extracted in step (A) by controlling supply of the eluent and the blocked position without supplying the feed solution while keeping the extraction position of the weakly adsorptive fraction as it is, and then shifting the extraction position of the weakly adsorptive fraction to a downstream side and extracting only the weakly adsorptive fraction, wherein, in step (B), at least one of the step α is performed.
[3] The simulated moving-bed type chromatographic separation method described in [2],
   wherein the pipes of the circulation system are provided with a feed solution supply port F, an eluent supply port D, a weakly adsorptive fraction extraction port A, an intermediately adsorptive fraction extraction port B, and a strongly adsorptive fraction extraction port C,
   wherein, in step (A), the feed solution supply port F, the extraction port A, the extraction port B, and the extraction port C are positioned in accordance with the following (a) to (c):
      (a) the extraction port A is provided downstream of the feed solution supply port F with at least one section interposed therebetween;
      (b) the extraction port B is provided on the pipe having the feed solution supply port F; and
      (c) the extraction port C is provided upstream of the extraction port B with at least one section interposed therebetween; and
   wherein, in step (B), the extraction port A and the extraction port C are positioned in accordance with the following (d):
      (d) the extraction port A is provided downstream of the extraction port C with at least two sections interposed therebetween.
[4] The simulated moving-bed type chromatographic separation method described in [2] or [3], wherein the number of times of step α performed in step (B) is [the number of sections -2] or [the number of sections] or more.
[5] The simulated moving-bed type chromatographic separation method described in any one of [2] to [4], including: using a circulation system in which four or more unit packed columns each filled with an adsorbent are connected in series and in an endless form via pipes, and dividing the circulation system into four sections annularly continuous from an upstream side to a downstream side so that each section has at least one of the unit packed column,
   wherein sub-steps (A1) to (A4) in step (A) are sub-steps (A1-1) to (A4-1) below, and
   wherein, in step (B), the following sub-steps (B1-1), (B2-1) and (B3-1) and sub-steps (B1'-1) (B2'-1) and (B3'-1) are performed in sequence:
      <Sub-step (A1-1)>
         supplying an eluent d-II from an eluent supply port D-II while using an upstream end of the section 1 as the eluent supply port D-II, extracting the intermediately adsorptive fraction from an intermediately adsorptive fraction extraction port B while using a downstream end of the section 2 as the extraction port B, supplying the feed solution from a feed solution supply port F while using an upstream end of the section 3 as the feed solution supply port F, and extracting the weakly adsorptive fraction from a weakly adsorptive fraction extraction port A while using a downstream end of the section 4 as the extraction port A,
         thereby most strengthening strongest desorption strength of the eluent passing through the sections 1 and 2, and weakening desorption strength of the eluent passing through the sections 3 and 4 more than the desorption strength of the eluent passing through the sections 1 and 2;
      <Sub-step (A2-1)>
         supplying an eluent d-II from an eluent supply port D-II while using an upstream end of the section 1 as the eluent supply port D-II, extracting the intermediately adsorptive fraction from an intermediately adsorptive fraction extraction port B while using a downstream end of the section 2 as the extraction port B, supplying an eluent d-IV from an eluent supply port D-IV while using an upstream end of the section 3 as the eluent supply port D-IV, and extracting the weakly adsorptive fraction from a weakly adsorptive fraction extraction port A while using a downstream end of the section 4 as the extraction port A,
         thereby most strengthening strongest desorption strength of the eluent passing through the sections 1 and 2, and weakening desorption strength of the eluent passing through the sections 3 and 4 more than the desorption strength of the eluent passing through the sections 1 and 2;
      <Sub-step (A3-1)>
         supplying an eluent d-I from an eluent supply port D-I while using an upstream end of the section 1 as the eluent supply port D-I, extracting the strongly adsorptive fraction from a strongly adsorptive fraction extraction port C while using a downstream end of the section 1 as the extraction port C, supplying an eluent d-II from an eluent supply port D-II while using an upstream end of the section 2 as the eluent supply port D-II, supplying an eluent d-III from an eluent supply port D-III while using an upstream end of the section 3 as the eluent supply port D-III, supplying an eluent d-IV from an eluent supply port D-IV while using an upstream end of the section 4 as the eluent supply port D-IV, and extracting the weakly adsorptive fraction from a weakly adsorptive fraction extraction port A while using a downstream end of the section 4 as the extraction port A,
         thereby most strengthening strongest desorption strength of the eluent passing through the section 1, weakening desorption strength of the eluent passing through the section 2 more than the desorption strength of the eluent passing through the section 1, weakening desorption strength of the eluent passing through the section 3 more than the desorption strength of the eluent passing through the section 2, and weakening desorption strength of the eluent passing through the section 4 more than the desorption strength of the eluent passing through the section 3;
      <Sub-step (A4-1)>
         supplying an eluent d-II from an eluent supply port D-II while using an upstream end of the section 2 as the eluent supply port D-II, supplying an eluent d-III from an eluent supply port D-III while using an upstream end of the section 3 as the eluent supply port D-III, supplying an eluent d-IV from an eluent supply port D-IV while using an upstream end of the section 4 as the eluent supply port D-IV, and extracting the weakly adsorptive fraction from a weakly adsorptive fraction extraction port A while using a downstream end of section 1 as the extraction port A,
         thereby most strengthening strongest desorption strength of the eluent passing through the section 2, weakening desorption strength of the eluent passing through the section 3 more than the desorption strength of the eluent passing through the section 2, weakening desorption strength of the eluent passing through the section 4 more than the desorption strength of the eluent passing through the section 3, and making the same desorption strength of the eluent passing through the section 1 as the desorption strength of the eluent passing through the section 4;
      <Sub-step (B1 -1)>
         supplying an eluent d-II from an eluent supply port D-II while using an upstream end of the section 2 as the eluent supply port D-II, supplying an eluent d-III from an eluent supply port D-III while using an upstream end of the section 4 as the eluent supply port D-III, supplying an eluent d-IV from an eluent supply port D-IV while using an upstream end of the section 1 as the eluent supply port D-IV, and extracting the weakly adsorptive fraction from a weakly adsorptive fraction extraction port A while using a downstream end of the section 1 as the extraction port A,
         thereby most strengthening strongest desorption strength of the eluent passing through the sections 2 and 3, weakening desorption strength of the eluent passing through the section 4 more than the desorption strength of the eluent passing through the sections 2 and 3, and weakening desorption strength of the eluent passing through the section 1 more than the desorption strength of the eluent passing through the section 4;
      <Sub-step (B2-1)>
         supplying an eluent d-I from an eluent supply port D-I while using an upstream end of the section 2 as the eluent supply port D-I, extracting the strongly adsorptive fraction from a strongly adsorptive fraction extraction port C while using a downstream end of the section 2 as the extraction port C, supplying an eluent d-II from an eluent supply port D-II while using an upstream end of the section 3 as the eluent supply port D-II, supplying an eluent d-III from an eluent supply port D-III while using an upstream end of the section 4 as the eluent supply port D-III, supplying an eluent d-IV from an eluent supply port D-IV while using an upstream end of the section 1 as the eluent supply port D-IV, and extracting the weakly adsorptive fraction from a weakly adsorptive fraction extraction port A while using a downstream end of the section 1 as the extraction port A,
         thereby most strengthening strongest desorption strength of the eluent passing through the section 2, weakening desorption strength of the eluent passing through the section 3 more than the desorption strength of the eluent passing through the section 2, weakening desorption strength of the eluent passing through the section 4 more than the desorption strength of the eluent passing through the section 3, and weakening desorption strength of the eluent passing through the section 1 more than the desorption strength of the eluent passing through the section 4;
      <Sub-step (B3-1)>
         supplying an eluent d-II from an eluent supply port D-II while using an upstream end of the section 3 as the eluent supply port D-II, supplying an eluent d-III from an eluent supply port D-III while using an upstream end of the section 4 as the eluent supply port D-III, supplying an eluent d-IV from an eluent supply port D-IV while using an upstream end of the section 1 as the eluent supply port D-IV, and extracting the weakly adsorptive fraction from a weakly adsorptive fraction extraction port A while using a downstream end of the section 2 as the extraction port A,
         thereby most strengthening strongest desorption strength of the eluent passing through the section 3, weakening desorption strength of the eluent passing through the section 4 more than the desorption strength of the eluent passing through the section 3, weakening desorption strength of the eluent passing through the section 1 more than the desorption strength of the eluent passing through the section 4, and making the same desorption strength of the eluent passing through the section 2 as the desorption strength of the eluent passing through the section 1;
      <Sub-step (B1'-1)>
         supplying an eluent d-II from an eluent supply port D-II while using an upstream end of the section 3 as the eluent supply port D-II, supplying an eluent d-III from an eluent supply port D-III while using an upstream end of the section 1 as the eluent supply port D-III, supplying an eluent d-IV from an eluent supply port D-IV while using an upstream end of the section 2 as the eluent supply port D-IV, and extracting the weakly adsorptive fraction from a weakly adsorptive fraction extraction port A while using a downstream end of the section 2 as the extraction port A,
         thereby most strengthening strongest desorption strength of the eluent passing through the sections 3 and 4, weakening desorption strength of the eluent passing through the section 1 more than the desorption strength of the eluent passing through the sections 3 and 4, and weakening desorption strength of the eluent passing through the section 2 more than the desorption strength of the eluent passing through the section 1;
      <Sub-step (B2'-1)>
         supplying an eluent d-I from an eluent supply port D-I while using an upstream end of the section 3 as the eluent supply port D-I, extracting the strongly adsorptive fraction from a strongly adsorptive fraction extraction port C while using a downstream end of the section 3 as the extraction port C, supplying an eluent d-II from an eluent supply port D-II while using an upstream end of the section 4 as the eluent supply port D-II, supplying an eluent d-III from an eluent supply port D-III while using an upstream end of the section 1 as the eluent supply port D-III, supplying an eluent d-IV from an eluent supply port D-IV while using an upstream end of the section 2 as the eluent supply port D-IV, and extracting the weakly adsorptive fraction from a weakly adsorptive fraction extraction port A while using a downstream end of the section 2 as the extraction port A,
         thereby most strengthening strongest desorption strength of the eluent passing through the section 3, weakening desorption strength of the eluent passing through the section 4 more than the desorption strength of the eluent passing through the section 3, weakening desorption strength of the eluent passing through the section 1 more than the desorption strength of the eluent passing through the section 4, and weakening desorption strength of the eluent passing through the section 2 more than the desorption strength of the eluent passing through the section 1; and
      <Sub-step (B3'-1)>
         supplying an eluent d-II from an eluent supply port D-II while using an upstream end of the section 4 as the eluent supply port D-II, supplying an eluent d-III from an eluent supply port D-III while using an upstream end of the section 1 as the eluent supply port D-III, supplying an eluent d-IV from an eluent supply port D-IV while using an upstream end of the section 2 as the eluent supply port D-IV, and extracting the weakly adsorptive fraction from a weakly adsorptive fraction extraction port A while using a downstream end of the section 3 as the extraction port A,
         thereby most strengthening strongest desorption strength of the eluent passing through the section 4, weakening desorption strength of the eluent passing through the section 1 more than the desorption strength of the eluent passing through the section 4, weakening desorption strength of the eluent passing through the section 2 more than the desorption strength of the eluent passing through the section 1, and making the same desorption strength of the eluent passing through the section 3 as the desorption strength of the eluent passing through the section 2.
[6] The simulated moving-bed type chromatographic separation method described in any one of [2] to [4], including: using a circulation system in which three or more unit packed columns each filled with an adsorbent are connected in series and in an endless form via pipes, and dividing the circulation system into three sections annularly continuous from an upstream side to a downstream side so that each section has at least one of the unit packed column,
   wherein sub-steps (A1) to (A4) in step (A) are sub-steps (A1-2) to (A4-2) below, and
   wherein, in step (B), the following sub-steps (B1-2) and (B2-2) are performed in sequence:
      <Sub-step (A1-2)>
         supplying an eluent d-II from an eluent supply port D-II while using an upstream end of the section 1 as the eluent supply port D-II, extracting the intermediately adsorptive fraction from an intermediately adsorptive fraction extraction port B while using a downstream end of the section 2 as the extraction port B, supplying the feed solution from a feed solution supply port F while using an upstream end of the section 3 as the feed solution supply port F, and extracting the weakly adsorptive fraction from a weakly adsorptive fraction extraction port A while using a downstream end of the section 3 as the extraction port A,
         thereby most strengthening strongest desorption strength of the eluent passing through the sections 1 and 2, and weakening desorption strength of the eluent passing through the section 3 more than the desorption strength of the eluent passing through the sections 1 and 2;
      <Sub-step (A2-2)>
         supplying an eluent d-II from an eluent supply port D-II while using an upstream end of the section 1 as the eluent supply port D-II, extracting the intermediately adsorptive fraction from an intermediately adsorptive fraction extraction port B while using a downstream end of the section 2 as the extraction port B, supplying an eluent d-IV from an eluent supply port D-IV while using an upstream end of the section 3 as the eluent supply port D-IV, and extracting the weakly adsorptive fraction from a weakly adsorptive fraction extraction port A while using a downstream end of the section 3 as the extraction port A,
         thereby most strengthening strongest desorption strength of the eluent passing through the sections 1 and 2, and weakening desorption strength of the eluent passing through the section 3 more than the desorption strength of the eluent passing through the sections 1 and 2;
      <Sub-step (A3-2)>
         supplying an eluent d-I from an eluent supply port D-I while using an upstream end of the section 1 as the eluent supply port D-I, extracting the strongly adsorptive fraction from a strongly adsorptive fraction extraction port C while using a downstream end of the section 1 as the extraction port C, supplying an eluent d-II from an eluent supply port D-II while using an upstream end of the section 2 as the eluent supply port D-II, supplying an eluent d-III from an eluent supply port D-III while using an upstream end of the section 3 as the eluent supply port D-III, and extracting the weakly adsorptive fraction from a weakly adsorptive fraction extraction port A while using a downstream end of the section 3 as the extraction port A,
         thereby most strengthening strongest desorption strength of the eluent passing through the section 1, weakening desorption strength of the eluent passing through the section 2 more than the desorption strength of the eluent passing through the section 1, and weakening desorption strength of the eluent passing through the section 3 more than the desorption strength of the eluent passing through the section 2;
      <Sub-step (A4-2)>
         supplying an eluent d-II from an eluent supply port D-II while using an upstream end of the section 2 as the eluent supply port D-II, supplying an eluent d-III from an eluent supply port D-III while using an upstream end of the section 3 as the eluent supply port D-III, supplying an eluent d-IV from an eluent supply port D-IV while using an upstream end of the section 1 as the eluent supply port D-IV, and extracting the weakly adsorptive fraction from a weakly adsorptive fraction extraction port A while using a downstream end of the section 1 as the extraction port A,
         thereby most strengthening strongest desorption strength of the eluent passing through the section 2, weakening desorption strength of the eluent passing through the section 3 more than the desorption strength of the eluent passing through the section 2, and weakening desorption strength of the eluent passing through the section 1 more than the desorption strength of the eluent passing through the section 3;
      <Sub-step (B1-2)>
         supplying an eluent d-I from an eluent supply port D-I while using an upstream end of the section 2 as the eluent supply port D-I, extracting the strongly adsorptive fraction from a strongly adsorptive fraction extraction port C while using a downstream end of the section 2 as the extraction port C, supplying an eluent d-II from an eluent supply port D-II while using an upstream end of the section 3 as the eluent supply port D-II, supplying an eluent d-III from an eluent supply port D-III while using an upstream end of the section 1 as the eluent supply port D-III, and extracting the weakly adsorptive fraction from a weakly adsorptive fraction extraction port A while using a downstream end of the section 1 as the extraction port A,
         thereby most strengthening strongest desorption strength of the eluent passing through the section 2, weakening desorption strength of the eluent passing through the section 3 more than the desorption strength of the eluent passing through the section 2, and weakening desorption strength of the eluent passing through the section 1 more than the desorption strength of the eluent passing through the section 3; and
      <Sub-step (B2-2)>
         supplying an eluent d-II from an eluent supply port D-II while using an upstream end of the section 3 as the eluent supply port D-II, supplying an eluent d-III from an eluent supply port D-III while using an upstream end of the section 1 as the eluent supply port D-III, supplying an eluent d-IV from an eluent supply port D-IV while using an upstream end of the section 2 as the eluent supply port D-IV, and extracting the weakly adsorptive fraction from a weakly adsorptive fraction extraction port A while using a downstream end of the section 2 as the extraction port A,
         thereby most strengthening strongest desorption strength of the eluent passing through the section 3, weakening desorption strength of the eluent passing through the section 1 more than the desorption strength of the eluent passing through the section 3, and weakening desorption strength of the eluent passing through the section 2 more than the desorption strength of the eluent passing through the section 1.
[7] The simulated moving-bed type chromatographic separation method described in any one of [1] to [6], wherein the intermediately adsorptive component is a biopolymer.
[8] A simulated moving-bed type chromatographic separation system obtained by using a circulation system in which three or more unit packed columns each filled with an adsorbent are connected in series and in an endless form via pipes, dividing the circulation system into at least three sections annularly continuous from an upstream side to a downstream side so that each section has at least one of the unit packed column, and separating, by using an eluent, a weakly adsorptive component, a strongly adsorptive component, and an intermediately adsorptive component having adsorption performance on the adsorbent, which performance is intermediate between the previous two components, the weakly adsorptive component, strongly adsorptive component, and intermediately adsorptive component being included in a feed solution, the system including means for repeating a cycle containing a first process and a second process below:
   <first process>
      a process containing blocking circulation of the circulation system by setting a blocked position to a position upstream of the weakly adsorptive component-rich section, supplying the feed solution to a section downstream of the blocked position, and extracting, from the circulation system, the intermediately adsorptive component-rich intermediately adsorptive fraction from a section upstream of the blocked position; and
   <second process>
      a process containing extracting the strongly adsorptive component-rich strongly adsorptive fraction by controlling supply of the eluent and blocking of the circulation system without supplying the feed solution, and shifting a supply position of the eluent relative to the circulation system and an extraction position of the strongly adsorptive fraction to a downstream side in accordance with movement of the strongly adsorptive fraction, and
   wherein in the cycle containing the first process and the second process, the extraction position of the strongly adsorptive fraction is shifted to a downstream side by 2 sections or more and the [number of sections] shifted toward the downstream side and the total number of sections are not the same.
[9] A simulated moving-bed type chromatographic separation system obtained by using a circulation system in which three or more unit packed columns each filled with an adsorbent are connected in series and in an endless form via pipes, dividing the circulation system into at least three sections annularly continuous from an upstream side to a downstream side so that each section has at least one of the unit packed column, and separating, by using an eluent, a weakly adsorptive component, a strongly adsorptive component, and an intermediately adsorptive component having adsorption performance on the adsorbent, which performance is intermediate between the previous two components, the weakly adsorptive component, strongly adsorptive component, and intermediately adsorptive component being included in a feed solution, the system including means for repeating a cycle containing step (A) and step (B) below:
   <step (A)>
      a step including:
      sub-step (A1) of blocking circulation of the circulation system by setting a blocked position to a position upstream of the weakly adsorptive component-rich section, supplying the feed solution at a section downstream of the blocked position and extracting, from the circulation system, the weakly adsorptive component-rich weakly adsorptive fraction at a position further downstream of the blocked position, and supplying an eluent for desorbing the intermediately adsorptive component at a section upstream of the blocked position and extracting, from the circulation system, the intermediately adsorptive component-rich intermediately adsorptive fraction at a position downstream thereof,
      sub-step (A2) of supplying an eluent for desorbing the weakly adsorptive component instead of supplying the feed solution and continuing extraction of the weakly adsorptive fraction and extraction of the intermediately adsorptive fraction,
      sub-step (A3) of shifting the blocked position to an upstream side and supplying an eluent for desorbing the strongly adsorptive component at a position upstream of the blocked position having been shifted and extracting the strongly adsorptive component-rich strongly adsorptive fraction at a position further downstream thereof while continuing extraction of the weakly adsorptive fraction, and
      sub-step (A4) of shifting the extraction position of the weakly adsorptive fraction to a downstream side and extracting the weakly adsorptive fraction while extracting neither the intermediately adsorptive fraction nor the strongly adsorptive fraction,
      wherein, in step (A), the sub-steps (A1), (A2), (A3) and (A4) are performed in this sequence; and
   <step (B)>
      a step including step α of separately extracting each of the weakly adsorptive fraction or strongly adsorptive fraction not extracted in step (A) by controlling supply of the eluent and the blocked position without supplying the feed solution while keeping the extraction position of the weakly adsorptive fraction as it is, and then shifting the extraction position of the weakly adsorptive fraction to a downstream side and extracting only the weakly adsorptive fraction, wherein, in step (B), at least one of the step α is performed.

In the present specification, the terms "upstream" and "downstream" are used with respect to the flow direction of fluid in the circulation system. That is, the term "upstream side" with respect to a certain part in the circulation system means a side on which the fluid flows toward the part, and the term "downstream side" means a side on which the fluid flows out from the part.

In the present specification, the term "strongly adsorptive component" means a component having strong adsorption strength with respect to an adsorbent for a plurality of components contained in a feed solution, the term "weakly adsorptive component" means a component having weak adsorption strength with respect to the adsorbent for the plurality of components contained in the feed solution, and the term "intermediately adsorptive component" means a component having weaker adsorption strength with respect to the adsorbent than the strongly adsorptive component and having stronger adsorption strength with respect to the adsorbent than the weakly adsorptive component. That is, the terms "strongly adsorptive", "intermediately adsorptive", and "weakly adsorptive" indicate the relative adsorption strength when comparing adsorption strength of each component contained in the feed solution with respect to the adsorbent.

The above-described "strongly adsorptive component", "intermediately adsorptive component", and "weakly adsorptive component" may each be composed of a single component or a plurality of components. Furthermore, the plurality of components may have the same or different adsorption strength.

Grouping of respective components in the feed solution into the "strongly adsorptive component", the "intermediately adsorptive component", and the "weakly adsorptive component" can be appropriately set depending on purposes. When a case where the feed solution contains four kinds of components is used as an example, two kinds of components in descending order according to adsorption strength with respect to the adsorbent can be collectively regarded as the strongly adsorptive component, a component having the third strongest adsorption strength with respect to the adsorbent can be regarded as the intermediately adsorptive component, and a component having the weakest adsorption strength with respect to the adsorbent can be regarded as the weakly adsorptive component. Furthermore, a component having the strongest adsorption strength with respect to the adsorbent can also be regarded as the strongly adsorptive component, a component having the second strongest adsorption strength and a component having the third strongest adsorption strength with respect to the adsorbent can also be collectively regarded as the intermediately adsorptive component, and a component having the weakest adsorption strength with respect to the adsorbent can also be regarded as the weakly adsorptive component. Furthermore, a component having the strongest adsorption strength with respect to the adsorbent can also be regarded as the strongly adsorptive component, a component having the second strongest adsorption strength with respect to the adsorbent can also be regarded as the intermediately adsorptive component, and a component having the third strongest adsorption strength with respect to the adsorbent and a component having the weakest adsorption strength with respect to the adsorbent can also be collectively regarded as the weakly adsorptive component. The same applies to a case where the feed solution contains five or more kinds of components. They can be separated and purified on the basis of such grouping.

In the present invention, the term "desorption strength" of the eluent means a strength of effect of desorbing a component adsorbed on an adsorbent from the adsorbent.

### ADVANTAGEOUS EFFECTS OF INVENTION

The simulated moving-bed type chromatographic separation method of the present invention makes it possible to fractionate a purification target component in a feed solution at high purity.

### BRIEF DESCRIPTION OF DRAWINGS

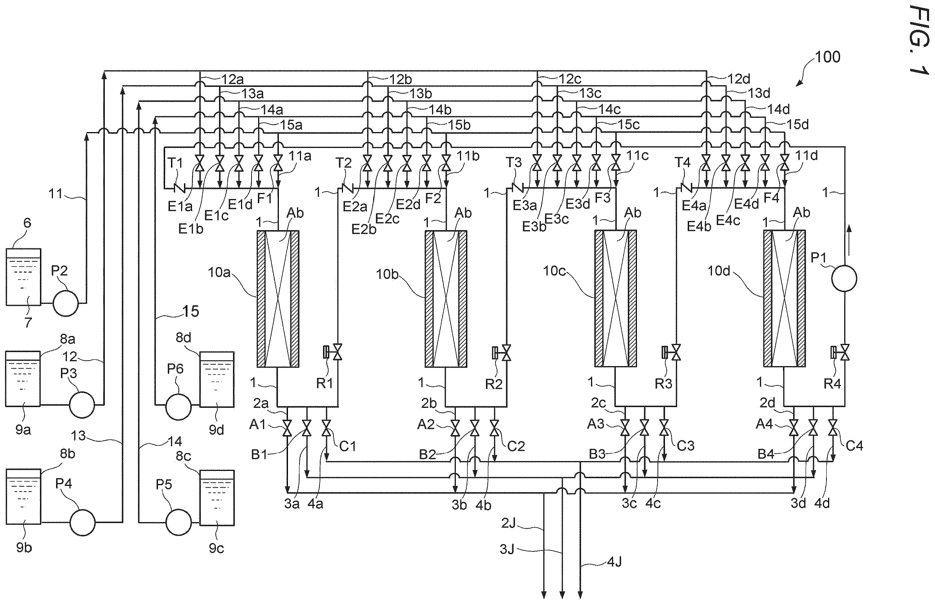

{FIG. 1}
   FIG. 1 is a system diagram illustrating an example of a simulated moving-bed type chromatographic separation system of the present invention.
{FIG. 2}
   FIG. 2 is a flow diagram of respective sub-steps constituting steps (A) and (B) in an embodiment of the simulated moving-bed type chromatographic separation method of the present invention.
{FIG. 3}
   FIG. 3 is a flow diagram of respective sub-steps constituting steps (A) and (B) in the next cycle after steps (A) and (B) shown in FIG. 2 are ended.
{FIG. 4}
   FIG. 4 is a flow diagram of respective sub-steps constituting steps (A) and (B) in another embodiment of the simulated moving-bed type chromatographic separation method of the present invention.
{FIG. 5}
   FIG. 5 is a flow diagram of respective sub-steps constituting steps (A) and (B) in the next cycle after steps (A) and (B) shown in FIG. 4 are ended.

Preferred embodiments of a simulated moving-bed type chromatographic separation method of the present invention (hereinafter, also simply referred to as the "method of the present invention") will be described.

The method of the present invention is carried out by using a circulation system in which a plurality of unit packed columns each packed with an adsorbent are connected in series and in an endless form via pipes. The circulation system itself used in the simulated moving-bed scheme is publicly known, and for example, JP-A-2009-36536, Japanese Patent No. 4606092 B2, and the like can be referred to.

The circulation system will be described below using the drawings; however, the present invention is not limited to these embodiments, except for the requirements defined by the present invention.

Incidentally, the drawings described below are explanatory diagrams for facilitating understanding of the present invention, and regarding sizes and relative magnitude relationships of respective configurations, the large one or the small one is sometimes changed for the purpose of illustration, and the form does not show a real relation as it is. Furthermore, the present invention is not limited to shapes, relative positional relationships, and the like illustrated in these drawings, except for the requirements defined by the present invention.

Furthermore, conditions other than the requirements defined by the present invention, such as the capacity of a unit packed column, intratubular cross-sectional areas and lengths of pipes, and a flow rate of a liquid to be supplied to the circulation system, can be appropriately set depending on purposes.

A preferred embodiment of a circulation system used in the method of the present invention is illustrated in FIG. 1. A circulation system 100 illustrated in FIG. 1 includes four unit packed columns (columns) (unit packed columns 10a, 10b, 10c, and 10d) packed with an adsorbent Ab, an exit of each unit packed column is connected to an entrance of an adjacent unit packed column via a pipe 1, and respective unit packed columns are connected in series as a whole.

Further, all of the unit packed columns are connected in an endless form (in an annular form) by connecting the exit of a most downstream-side unit packed column (for example, the unit packed column 10d) to the entrance of a most upstream-side unit packed column (for example, the unit packed column 10a) via the pipe 1. With such a configuration, a fluid can be circulated in the circulation system 100. The unit packed columns 10a to 10d may be the same as or different from each other in the inner shape, the size, and the amount of the adsorbent packed. It is preferable to use the unit packed columns 10a to 10d of which the inner shape, size, and the amount of the adsorbent packed are all equivalent (preferably are the same).

A circulation pump P1 for allowing a fluid to flow in an arrow direction can be arranged in the circulation system 100. The circulation pump P1 is preferably a metering pump. Furthermore, in the circulation system 100, shutoff valves R1, R2, R3, and R4 capable of shutting off the circulation of the fluid to the unit packed column on the downstream side of the pipe 1 are provided on the pipe 1 between two adjacent unit packed columns.

Weakly adsorptive fraction extracting lines 2a, 2b, 2c, and 2d for extracting a fraction containing much weakly adsorptive components with respect to the adsorbent Ab (referred to as "weakly adsorptive fraction with respect to the adsorbent Ab" or simply referred to as "weakly adsorptive fraction" in the present specification) are divergently arranged between the respective shutoff valves R1 to R4 and the exits of the respective unit packed columns 10a to 10d positioned at the upstream sides thereof, respectively. Weakly adsorptive fraction extracting valves A1, A2, A3, and A4 capable of opening and closing the respective weakly adsorptive fraction extracting lines are provided on the respective weakly adsorptive fraction extracting lines 2a, 2b, 2c, and 2d, respectively. The respective weakly adsorptive fraction extracting lines 2a, 2b, 2c, and 2d are joined into a single weakly adsorptive fraction joining pipe 2J.

Furthermore, similarly, intermediately adsorptive fraction extracting lines 3a, 3b, 3c, and 3d for extracting a fraction containing much intermediately adsorptive components with respect to the adsorbent Ab (referred to as "intermediately adsorptive fraction with respect to the adsorbent Ab" or simply referred to as "intermediately adsorptive fraction" in the present specification) are divergently arranged between the respective shutoff valves R1 to R4 and the exits of the respective unit packed columns 10a to 10d positioned at the upstream sides thereof. Intermediately adsorptive fraction extracting valves B1, B2, B3, and B4 capable of opening and closing the respective intermediately adsorptive fraction extracting lines are provided on the respective intermediately adsorptive fraction extracting lines 3a, 3b, 3c, and 3d, respectively. The respective intermediately adsorptive fraction extracting lines 3a, 3b, 3c, and 3d are joined into a single intermediately adsorptive fraction joining pipe 3J.

Furthermore, similarly, strongly adsorptive fraction extracting lines 4a, 4b, 4c, and 4d for extracting a fraction containing much strongly adsorptive components with respect to the adsorbent Ab (referred to as "strongly adsorptive fraction with respect to the adsorbent Ab" or simply referred to as "strongly adsorptive fraction" in the present specification) are divergently arranged between the respective shutoff valves R1 to R4 and the exits of the respective unit packed columns 10a to 10d positioned at the upstream sides thereof. Strongly adsorptive fraction extracting valves C1, C2, C3, and C4 capable of opening and closing the respective strongly adsorptive fraction extracting lines are provided on the respective strongly adsorptive fraction extracting lines 4a, 4b, 4c, and 4d, respectively. The respective strongly adsorptive fraction extracting lines 4a, 4b, 4c, and 4d are joined into a single strongly adsorptive fraction joining pipe 4J.

In the processes and steps described below, some of the weakly adsorptive fraction extracting valves A1, A2, A3, and A4 are in a state of being opened. A connection part between the weakly adsorptive fraction extracting line in which the opened weakly adsorptive fraction extracting valve is provided and the pipe 1 becomes an extraction port A of a weakly adsorptive fraction in the processes and steps described below.

Furthermore, in the processes and steps described below, some of the intermediately adsorptive fraction extracting valves B1, B2, B3, and B4 are in a state of being opened. A connection part between the intermediately adsorptive fraction extracting line in which the opened intermediately adsorptive fraction extracting valve is provided and the pipe 1 becomes an extraction port B of an intermediately adsorptive fraction in the processes and steps described below.

Furthermore, in the processes and steps described below, some of the strongly adsorptive fraction extracting valves C1, C2, C3, and C4 are in a state of being opened. A connection part between the strongly adsorptive fraction extracting line in which the opened strongly adsorptive fraction extracting valve is provided and the pipe 1 becomes an extraction port C of a strongly adsorptive fraction in the processes and steps described below.

A safety valve (or a relief valve) (not illustrated) can be provided in an appropriate part in the circulation system 100 in order to prevent the pressure of the circulation system 100 from being excessively increased. Furthermore, check valves T1, T2, T3, and T4 for backflow prevention are preferably provided between two adjacent unit packed columns.

As illustrated in FIG. 1, the inside of the circulation system 100 is configured to capable of supplying a feed solution 7 housed in a feed solution tank 6. Further, the inside of the circulation system 100 is configured to be capable of supplying three or more kinds of eluents. In FIG. 1, as an example, an embodiment in which four kinds of eluents are supplied is illustrated.

The feed solution 7 is supplied by a feed solution supply pump P2 capable of controlling the supply flow rate via a feed solution supplying line 11. The feed solution supply pump P2 is preferably a metering pump. The feed solution supplying line 11 is diverged into four divergent feed solution supplying lines 11a, 11b, 11c, and 11d as illustrated in FIG. 1, and is configured to capable of supplying the feed solution to the entrances of the respective unit packed columns 10a, 10b, 10c, and 10d via the respective divergent feed solution supplying lines 11a, 11b, 11c, and 11d. Feed solution supplying valves F1, F2, F3, and F4 capable of being opened/closed are provided in the respective divergent feed solution supplying lines 11a, 11b, 11c, and 11d, and the feed solution is supplied through a divergent feed solution supplying line having an opened feed solution supplying valve to a unit packed column connected to the downstream thereof.

In the processes and steps described below, some of the feed solution supplying valves F1, F2, F3, and F4 are in a state of being opened. A connection part between the divergent feed solution supplying line in which the opened feed solution supplying valve is provided and the pipe 1 becomes a feed solution supply port F in the processes and steps described below.

FIG. 1 illustrates an embodiment in which four kinds of eluents each having different desorption strength are supplied. An eluent 9a housed in an eluent tank 8a is supplied to an eluent supplying line 12 by an eluent supply pump P3 capable of controlling the supply flow rate. An eluent 9b housed in an eluent tank 8b is supplied to an eluent supplying line 13 by an eluent supply pump P4 capable of controlling the supply flow rate. An eluent 9c housed in an eluent tank 8c is supplied to an eluent supplying line 14 by an eluent supply pump P5 capable of controlling the supply flow rate. Further, an eluent 9d housed in an eluent tank 8d is supplied to an eluent supplying line 15 by an eluent supply pump P6 capable of controlling the supply flow rate.

The eluent supply pumps P3 to P6 are each preferably a metering pump. The eluent supplying line 12 is diverged into four divergent eluent supplying lines 12a, 12b, 12c, and 12d as illustrated in FIG. 1, and is configured to be capable of supplying the eluents to the entrances of the respective unit packed columns 10a, 10b, 10c, and 10d via the respective divergent eluent supplying lines 12a, 12b, 12c, and 12d. Eluent supplying valves E1a, E2a, E3a, and E4a capable of being opened/closed are provided in the respective divergent eluent supplying lines 12a, 12b, 12c, and 12d, and the eluent is supplied through a divergent eluent supplying line having an opened eluent supplying valve to a unit packed column connected to the downstream thereof.

Similarly, the eluent supplying line 13 is diverged into four divergent eluent supplying lines 13a, 13b, 13c, and 13d, the eluent supplying line 14 is diverged into four divergent eluent supplying lines 14a, 14b, 14c, and 14d, the eluent supplying line 15 is diverged into four divergent eluent supplying lines 15a, 15b, 15c, and 15d, and each eluent supplying line is configured to capable of supplying each eluent to the entrances of the respective unit packed columns 10a, 10b, 10c, and 10d.

Eluent supplying valves E1b, E2b, E3b, and E4b capable of being opened/closed are provided in the divergent eluent supplying lines 13a, 13b, 13c, 13d, eluent supplying valves E1c, E2c, E3c, and E4c capable of being opened/closed are provided in the divergent eluent supplying lines 14a, 14b, 14c, and 14d, and eluent supplying valves E1d, E2d, E3d, and E4d capable of being opened/closed are provided in the divergent eluent supplying lines 15a, 15b, 15c, and 15d, respectively.

In the processes and steps described below, a connection part between the divergent eluent supplying line in which the opened eluent supplying valve is provided and the pipe 1 becomes an eluent supply port D. In the processes and steps described below, there are a plurality of eluent supplying valves to be opened. Specifically, in the processes and steps described below, the number of eluent supply ports D present is the number corresponding to the kinds of eluent to be used.

Subsequently, the operation of the circulation system when the method of the present invention is carried out by the circulation system will be described; however, the present invention is not limited to these embodiments, except for the requirements defined by the present invention.

In the method of the present invention, the circulation system is provided such that three or more unit packed columns each packed with an adsorbent are connected in series and in an endless form via pipes. In addition, the circulation system is divided into at least three sections that are annularly continuous from the upstream side to the downstream side such that each section has at least one of the unit packed column. That is, one section may have a plurality of unit packed columns.

In the method of the present invention, a cycle containing a first process and a second process described later or a cycle containing step (A) and step (B) is repeated to be able to separate, by using an eluent(s), a weakly adsorptive component, a strongly adsorptive component, and an intermediately adsorptive component having adsorption performance on the adsorbent, which performance is intermediate between the previous two components, the weakly adsorptive component, strongly adsorptive component, and intermediately adsorptive component being included in a feed solution. In the first process and the second process described later, the characteristic operation of the present invention is specified mainly by the shifting of the extraction position of the strongly adsorptive component, and in step (A) and step (B) described later, the characteristic operation of the present invention is specified mainly by the shifting of the extraction position of the weakly adsorptive component.

In the present invention or the present specification, "repeating the cycle containing the first process and the second process" or "repeating the cycle containing step (A) and step (B)" means that after the end of the second process or step (B) (after the end of one cycle), the cycle is repeated by setting a position upstream of the weakly adsorptive component-rich section (preferably, an upstream end of the weakly adsorptive component-rich section) as the blocked position and newly starting to supply the feed solution to a section downstream of the blocked position in the first process or step (A).

In addition, in the present invention or the present specification, as an embodiment in which "the cycle containing step (A) and step (B) is repeated", after the end of step (B) (after the end of one cycle), the position where the weakly adsorptive fraction is extracted is kept as it is, and the position where the feed solution is supplied, the position where the eluent for desorbing the intermediately adsorptive component is supplied, and the position where the intermediately adsorptive component-rich intermediately adsorptive fraction is extracted are defined as in step (A) (sub-step (A1)), and step (A) of the next cycle can be started. That is, as described later, when the weakly adsorptive fraction extraction port and the feed solution supply port are constructed to be separated by one section in the sub-step (A1) of the previous cycle, the weakly adsorptive fraction extraction port and the feed solution supply port are constructed to be separated by one section even in the sub-step (A1) of the next cycle regardless of the position of the weakly adsorptive fraction extraction port.

In the method of the present invention, the sum of the amount of the feed solution to be supplied and the amount of the eluent to be supplied coincides with the sum of the amount of the weakly adsorptive fraction, the amount of the intermediately adsorptive fraction, and the amount of the strongly adsorptive fraction to be extracted. That is, in a state where the liquid is supplied into the circulation system, the same amount of the liquid as the amount of the liquid supplied is extracted from the inside of the circulation system.

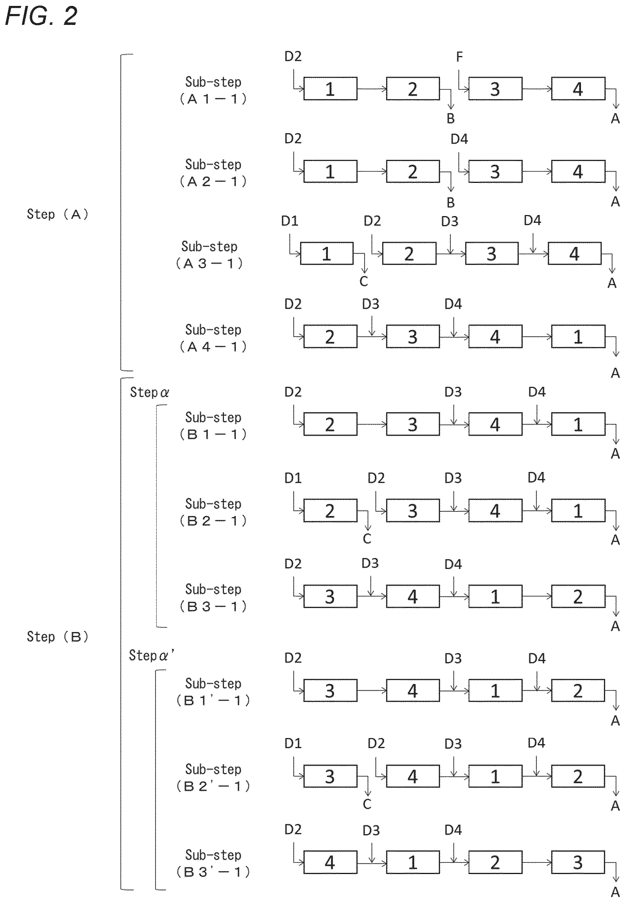

More specifically, in a case where a liquid is supplied from a certain supply port (X) and a liquid is extracted from an extraction port (Y) on the downstream side thereof, when the liquid is not supplied from the downstream side of X and the upstream side of Y, the amount of the liquid extracted from Y is the same as the amount of the liquid supplied from X. Furthermore, when the liquid is supplied from the downstream side of X and the upstream side of Y, the amount of the liquid extracted from Y is the same as the total of the amount of the liquid supplied from X and the amount of the liquid supplied from the downstream side of X and the upstream side of Y. For example, in the sub-step (A1-1) of FIG. 2, the amount of the eluent supplied from the eluent supply port D2 is the same as the amount of the intermediately adsorptive fraction extracted from the intermediately adsorptive fraction extraction port B. Also, in the sub-step (A1-1), the amount of the feed solution supplied from the feed solution supply port F is the same as the amount of the weakly adsorptive fraction extracted from the weakly adsorptive fraction extraction port A.

In the method of the present invention, it is preferable to use three or more kinds of eluents each having different desorption strength, further preferable to use three to six kinds of eluents each having different desorption strength, and particularly preferable to use three or four kinds of eluents each having different desorption strength.

The type of eluents is not particularly limited and is appropriately set by a relationship with the type of adsorbents or the type of components in a feed solution. For example, in the case of using an ion-exchange resin as an adsorbent, a plurality of eluents each having different desorption strength can be prepared by changing the salt concentration of the eluent. For example, in the case of using a cation-exchange resin as the adsorbent, a plurality of eluents having varied NaCl concentrations can be used.

Note that the following first process and second process, or the following step (A) and step (B) defined by the method of the present invention do not specify the first run (first cycle) of the first process and second process or step (A) and step (B), but assume a steady state after the first process and second process or step (A) and step (B) are sequentially performed at least once (preferably 2 or more times, more preferably 3 or more times, still more preferably 4 or more times, and still more preferably 5 or more times). For example, as in the flow diagram shown in FIG. 2, in the first run (first cycle), no intermediately adsorptive component is present in sections 1 and 2 since the feed solution is supplied from the upstream of section 3 in sub-step (A1-1). Thus, in the sub-step (A1 -1) in the first run, the intermediately adsorptive component-rich intermediately adsorptive fraction is not extracted from the intermediately adsorptive fraction extraction port B. However, by performing the following steps (A) and (B) defined by the method of the present invention at least once in this order, in the next cycle and the subsequent cycles, the intermediately adsorptive component-rich intermediately adsorptive fraction is extracted from the intermediately adsorptive fraction extraction port B in the sub-step (A1-1).

In an embodiment of the present invention, the operation of the circulation system characteristic of the present invention can be specified by setting a cycle containing the following first process and second process as one cycle:
<first process>
   a process containing blocking circulation of the circulation system by setting a blocked position to a position upstream of the weakly adsorptive component-rich section (preferably an upstream end of the weakly adsorptive component-rich section), supplying the feed solution to a section downstream of the blocked position, and extracting, from the circulation system, the intermediately adsorptive component-rich intermediately adsorptive fraction from a section upstream of the blocked position; and
<second process>
   a process containing extracting the strongly adsorptive component-rich strongly adsorptive fraction by controlling supply of the eluent and blocking of the circulation system without supplying the feed solution, and shifting a supply position of the eluent relative to the circulation system and an extraction position of the strongly adsorptive fraction to a downstream side in accordance with movement of the strongly adsorptive fraction.

The cycle in the present invention may be specified by the first process and the second process. In this cycle, the extraction position of the strongly adsorptive fraction is shifted to a downstream side by 2 sections or more and the [number of sections] shifted toward the downstream side and the total number of sections are not the same. The number of sections is the total number of sections of the circulation system. For example, in the method of the present invention, when the number of sections used in the circulation system may be 4, in one cycle, the strongly adsorptive fraction extraction position is shifted to the downstream side by 2 or 3 sections or by 5 or more sections.

While a position upstream of the weakly adsorptive component-rich section in the immediately prior process (preferably an upstream end of the weakly adsorptive component-rich section) is set to a blocked position, the blocked position is used as a reference to construct the position where the feed solution is supplied in the first process, the position where the eluent for desorbing the intermediately adsorptive component is supplied, and the position where the intermediately adsorptive component-rich intermediately adsorptive fraction is extracted. In the first cycle, an arbitrary position can be set as the blocked position.

In the first process and the second process, it is preferable to extract, at all times, the weakly adsorptive fraction from the weakly adsorptive component-rich section. As for the weakly adsorptive fraction extraction position, the description of the weakly adsorptive fraction extraction position in step (A) and step (B) described later can also be cited.

In an embodiment of the present invention, the operation of the circulation system characteristic of the present invention can be specified by setting a cycle containing the following step (A) and step (B) as one cycle.

### <Step (A)>

In step (A), the following sub-steps (A1) to (A4) are performed in this order:
sub-step (A1) of blocking circulation of the circulation system by setting a blocked position to a position upstream of the weakly adsorptive component-rich section (preferably an upstream end of the weakly adsorptive fraction-rich section), supplying the feed solution at a section downstream of the blocked position and extracting, from the circulation system, the weakly adsorptive component-rich weakly adsorptive fraction at a position further downstream of the blocked position, and supplying an eluent for desorbing the intermediately adsorptive component at a section upstream of the blocked position and extracting, from the circulation system, the intermediately adsorptive component-rich intermediately adsorptive fraction at a position downstream thereof,
sub-step (A2) of supplying an eluent for desorbing the weakly adsorptive component instead of supplying the feed solution and continuing extraction of the weakly adsorptive fraction and extraction of the intermediately adsorptive fraction,
sub-step (A3) of shifting the blocked position to an upstream side and supplying an eluent for desorbing the strongly adsorptive component at a position upstream of the blocked position having been shifted and extracting the strongly adsorptive component-rich strongly adsorptive fraction at a position further downstream thereof while continuing extraction of the weakly adsorptive fraction, and
sub-step (A4) of shifting the extraction position of the weakly adsorptive fraction to a downstream side and extracting the weakly adsorptive fraction while extracting neither the intermediately adsorptive fraction nor the strongly adsorptive fraction.

In the present invention, the sub-steps (A1), (A2), (A3) and (A4) are performed in step (A) as described above.

In the present invention, "In step (A), sub-steps W, X, Y, and Z are performed" means that step (A) includes sub-steps W, X, Y, and Z. In addition, step (A) may include another sub-step(s) other than the sub-steps W, X, Y, and Z.

As an embodiment in which "the sub-steps W, X, Y, and Z are performed in step (A)", typically, an embodiment in which the sub-steps W, X, Y, and Z are performed in this order as step (A) is exemplified, but the present invention is not limited to this embodiment. That is, the expression "the sub-steps W, X, Y, and Z are performed in step (A)" is not limited to an embodiment in which the sub-step X is performed immediately after the sub-step W, the sub-step Y is performed immediately after the sub-step X, the sub-step Z is performed immediately after the sub-step Y, step (B) is performed immediately after the sub-step Z, and the sub-step W is performed immediately after step (B). For example, in the embodiment including other sub-steps, the other sub-steps (sub-steps other than the sub-steps W, X, Y and Z) may be incorporated at least one of before the sub-step W (between step (B) and the sub-step W), between the sub-steps W and X, between the sub-steps X and Y, between the sub-steps Y and Z, and between the sub-step Z and step (B) to the extent that the effects of the present invention are not impaired. There is a case where a target effect is obtainable even by incorporating additional sub-steps other than the sub-steps W, X, Y, and Z by adjustment of a supply flow rate, a flow rate, eluent strength, or the like, and this can be reasonably understood by those skilled in the art who pertain to the present specification.

This also applies to a case where step (B) has a sub-step.

### <Sub-step (A1)>

In the sub-step (A1), while the eluent for desorbing the intermediately adsorptive component is supplied, the amount of the intermediately adsorptive fraction extracted from the downstream is the same as the supply amount of the eluent for desorbing the intermediately adsorptive component. In this case, it is preferable that at least two sections (unit packed columns) are arranged between the supply port of the eluent for desorbing the intermediately adsorptive component and the intermediately adsorptive fraction extraction port downstream thereof. In addition, it is preferable that there is no other supply port therebetween.

In the sub-step (A1), while the feed solution is supplied, an embodiment is preferable in which the amount of the weakly adsorptive fraction extracted from the downstream is the same as the amount of the feed solution supplied. In this case, at least one section (unit packed column) is arranged between the feed solution supply port and the weakly adsorptive fraction extraction port downstream thereof. In addition, it is preferable that there is no other supply port therebetween.

While a position upstream of the weakly adsorptive component-rich section in the immediately prior step (preferably an upstream end of the weakly adsorptive component-rich section) is set to a blocked position, the blocked position is used as a reference to construct the position where the feed solution is supplied in the sub-step (A1), the position where the eluent for desorbing the intermediately adsorptive component is supplied, and the position where the intermediately adsorptive component-rich intermediately adsorptive fraction is extracted. In addition, it can also be constructed based on the weakly adsorptive fraction extracting position in the immediately prior step. In the first cycle, an arbitrary position can be set as the blocked position.

The position where the feed solution is supplied is preferably provided upstream of the position where the weakly adsorptive fraction is extracted with at least one section interposed therebetween.

The position where the intermediately adsorptive component-rich intermediately adsorptive fraction is extracted is preferably provided on the same pipe at the position where the feed solution is supplied (feed solution supply port) and on the upstream side of the position where the feed solution is supplied. That is, it is preferable that no unit packed column is provided between the position where the intermediately adsorptive component-rich intermediately adsorptive fraction is extracted and the position where the feed solution is supplied.

The position where the eluent for desorbing the intermediately adsorptive component is supplied is preferably provided on the same pipe at the position where the weakly adsorptive fraction is extracted (weakly adsorptive fraction extraction port) and on the upstream side of the position where the weakly adsorptive fraction is extracted. That is, it is preferable that no unit packed column is provided between the position where the eluent for desorbing the intermediately adsorptive component is supplied and the position where the weakly adsorptive fraction is extracted.

### <Sub-step (A2)>

In the sub-step (A2), while the eluent for desorbing the weakly adsorptive component is supplied instead of the feed solution, the amount of the weakly adsorptive fraction extracted from the downstream is the same as the supply amount of the eluent for desorbing the weakly adsorptive component. In the embodiment of the system shown in FIG. 1, the supply port of the feed solution and the supply port of the eluent for desorbing the weakly adsorptive component are different supply ports provided on the same pipe. The position of the weakly adsorptive fraction extraction port is preferably the same as the position of the weakly adsorptive fraction extraction port in the sub-step (A1).

In the sub-step (A2), the extraction of the intermediately adsorptive fraction in the sub-step (A1) is continued. That is, the intermediately adsorptive fraction extraction condition in the sub-step (A2) can be substantially the same as the intermediately adsorptive fraction extraction condition in the sub-step (A1).

### <Sub-step (A3)>

In the sub-step (A3), it is preferable to provide a supply port for supplying an eluent for desorbing the strongly adsorptive component on the same pipe as that for the supply port of the eluent for desorbing the intermediately adsorptive component in the sub-steps (A1) and (A2).

In addition, it is preferable to supply, downstream of the blocked position shifted, an eluent for desorbing the intermediately adsorptive component. Further, it is preferable to supply the eluent for desorbing the weakly adsorptive component from the downstream side across one section from the supply port of the eluent for desorbing the intermediately adsorptive component.

The position where the strongly adsorptive component-rich strongly adsorptive fraction is extracted is preferably provided on the same pipe at the position of the supply port of the eluent for desorbing the intermediately adsorptive component and on the upstream side of the position where the eluent is supplied. In addition, the position of the weakly adsorptive fraction extraction port is preferably the same as the position of the weakly adsorptive fraction extraction port in the sub-step (A2).

### <Sub-step (A4)>

In the sub-step (A4), it is preferable to shift the position of the weakly adsorptive fraction extraction port to the downstream side by one section from the weakly adsorptive fraction extraction port in the sub-step (A3) while keeping the supply port of the eluent for desorbing the intermediately adsorptive component and the supply port of the eluent for desorbing the weakly adsorptive component in the sub-step (A3) as they are.

### <Step (B)>

Step (B) is a step including step α of separately extracting each of the weakly adsorptive fraction or strongly adsorptive fraction not extracted in step (A) by controlling supply of the eluent and the blocked position without supplying the feed solution while keeping the extraction position of the weakly adsorptive fraction as it is, and then shifting the extraction position of the weakly adsorptive fraction to a downstream side and extracting only the weakly adsorptive fraction, wherein at least one of the step α is performed.

In step (B), it is preferable not to extract the intermediately adsorptive fraction. On the other hand, with the movement of the intermediately adsorptive component in the circulation system, a supply port of the eluent for desorbing the intermediately adsorptive component can be set. That is, the position upstream of the intermediately adsorptive component-rich section (preferably, an upstream end of the intermediately adsorptive component-rich section) can be set as the supply position of the eluent for desorbing the intermediately adsorptive component.

In step α, it is preferable to provide the supply port of the eluent for desorbing the strongly adsorptive component on the same pipe as that for the supply port of the eluent for desorbing the intermediately adsorptive component at the end of the immediately prior step or the immediately prior step α. That is, for example, when step α is performed once, the supply port of the eluent for desorbing the strongly adsorptive component in step (B) is preferably provided on the same pipe as that for the supply port of the eluent for desorbing the intermediately adsorptive component at the end of step (A) which is the immediately prior step (sub-step (A4)). For example, when step α is performed twice, the supply port of the eluent for desorbing the strongly adsorptive component in the second step α is preferably provided on the same pipe as that for the supply port of the eluent for desorbing the intermediately adsorptive component at the end of the first step α.

While step (B) is conducted, it is preferable to extract the weakly adsorptive fraction at all times. Therefore, even in a case where step (B) is configured by a plurality of sub-steps, it is preferable to extract the weakly adsorptive fraction in the plurality of sub-steps at all times.

In step α, as described above, the weakly adsorptive fraction and the strongly adsorptive fraction are extracted separately, and then the extraction position of the weakly adsorptive fraction is shifted to the downstream side to extract only the weakly adsorptive fraction. Here, when the step α is repeated, that is, when the step α is performed a plurality of times, the supply position of each eluent and the extraction position of each fraction at the start of the next step α are shifted to the downstream side by at least one section while maintaining the relative positional relationship of each position relative to the supply position of each eluent and the extraction position of each fraction at the start of the previous step α.

That is, at the start of the step α at the time of transition from the sub-step (A4) to the step α, the extraction position of the weakly adsorptive fraction is the same as the extraction position of the weakly adsorptive fraction in the sub-step (A4). In the step α, in order to shift the extraction position of the weakly adsorptive fraction to the downstream side, at the start of the next step α, the weakly adsorptive fraction extraction position shifted to the downstream side in the previous step α (the extraction position of the weakly adsorptive fraction at the end of the previous step α) is directly used as the extraction position of the weakly adsorptive fraction. The supply position of each eluent and the extraction position of the strongly adsorptive fraction other than the extraction position of the weakly adsorptive fraction in the next step α are reconstructed according to the definition of the step α based on the extraction position of the weakly adsorptive fraction at the start of the next step α. Therefore, by repeating the step α, each position is shifted to the downstream side by at least one section.

With repeated steps (A) and (B) (repeated cycle), it is preferable that the position where the feed solution is supplied, the position where the eluent is supplied, and the position where each fraction is extracted are shifted upstream by one or more sections as compared with the previous cycle while maintaining the relative positions. In this way, the respective positions are kept while maintaining the relative positions in the previous cycle and the subsequent cycle. This makes it possible to avoid the feed solution from being supplied from the same supply port at all times, and also makes it possible to prevent the same supply port from being excessively contaminated by the feed solution. How many sections are shifted upstream when repeating the cycle can be determined by the number of sections and the number of repetitions of the step α.

In step (A), it is preferable to provide, in the pipes of the circulation system, the feed solution supply port F for supplying the feed solution, the extraction port A for extracting the weakly adsorptive fraction, the extraction port B for extracting the intermediately adsorptive fraction, and the extraction port C for extracting the strongly adsorptive fraction at positions satisfying the following (a) to (c). That is, in repetition of steps (A) and (B), the relative positional relationship between the feed solution supply port F, the weakly adsorptive fraction extraction port A, the intermediately adsorptive fraction extraction port B, and the strongly adsorptive fraction extraction port C in step (A) satisfies (a) to (c) at all times.

(a) The weakly adsorptive fraction extraction port A is provided on the downstream side of the feed solution supply port F with at least one section interposed therebetween.
(b) The intermediately adsorptive fraction extraction port B is provided on the pipe having the feed solution supply port F.

Herein, the expression "the intermediately adsorptive fraction extraction port B is provided on the pipe having the feed solution supply port F" means that no unit packed column is arranged between the intermediately adsorptive fraction extraction port B and the feed solution supply port F. Furthermore, in a case where "the intermediately adsorptive fraction extraction port B is provided on the pipe having the feed solution supply port F", the intermediately adsorptive fraction extraction port B is provided upstream in the same pipe than the feed solution supply port F. This is applicable to all of the relationships between the extraction ports and the supply ports provided on the same pipes. That is, in the circulation system, in a certain extraction port and a certain supply port are provided on the same pipe (in a case where an extraction port and a supply port are provided without a unit packed column interposed therebetween), the extraction port is provided upstream in the same pipe than the supply port. This is to prevent the supplied liquid from being extracted from the extraction port before the liquid reaches the unit packed column on the downstream thereof.

(c) The strongly adsorptive fraction extraction port C is provided on the upstream side of the intermediately adsorptive fraction extraction port B with at least one section interposed therebetween.

In step (B), it is preferable to provide, in the pipes of the circulation system, the extraction port A for extracting the weakly adsorptive fraction and the extraction port C for extracting the strongly adsorptive fraction at positions satisfying the following (d). That is, in repetition of steps (A) and (B), the relative positional relationship between the weakly adsorptive fraction extraction port A and the strongly adsorptive fraction extraction port C in step (B) satisfies (d) at all times.

(d) The weakly adsorptive fraction extraction port A is provided on the downstream side of the strongly adsorptive fraction extraction port C with at least two sections interposed therebetween.

In step (B), the strongly adsorptive fraction extraction port C is preferably provided on a pipe having a supply port of the eluent for desorbing the intermediately adsorptive component. That is, the strongly adsorptive fraction extraction port C is preferably provided on the upstream side of the same pipe than the supply port of the eluent for desorbing the intermediately adsorptive component.

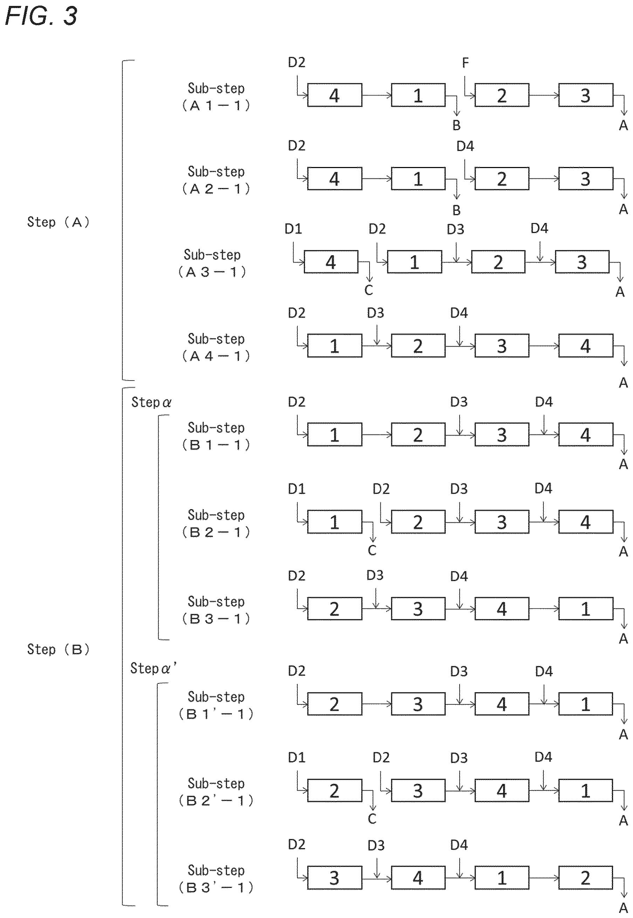

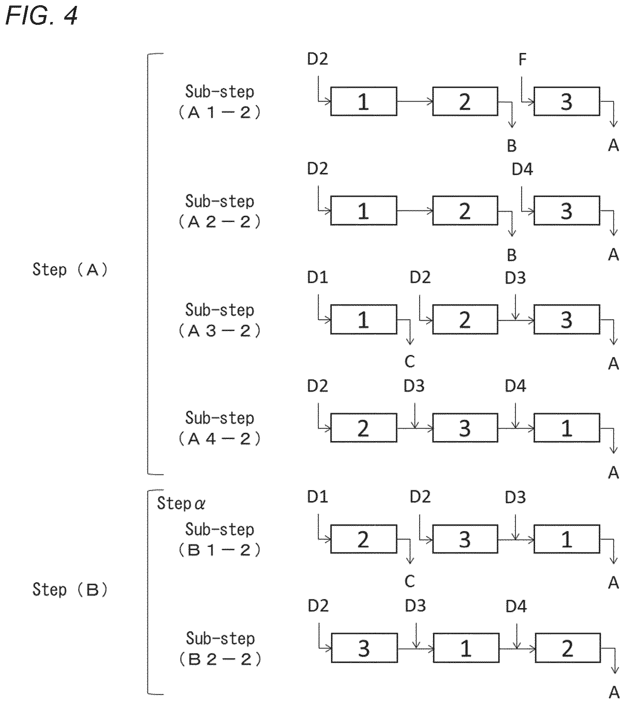

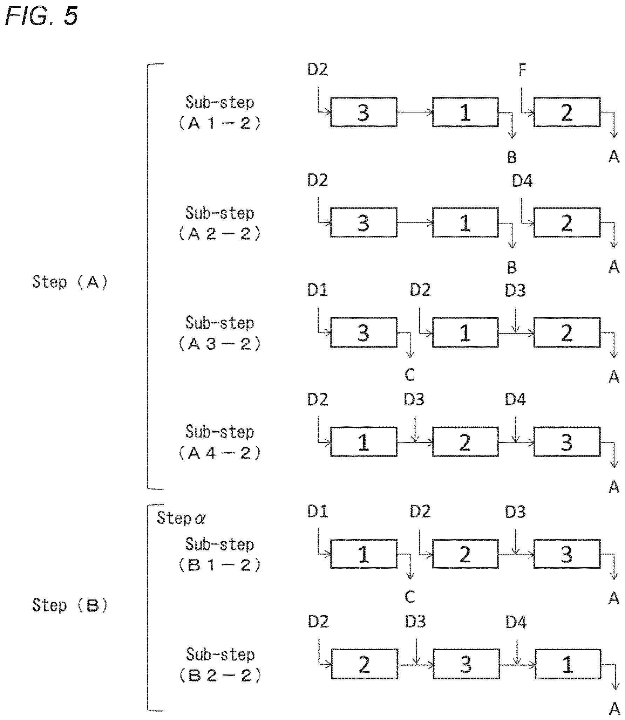

The number of times of step α performed in step (B) is [the number of sections -2] or [the number of sections] or more. The number of sections is the total number of sections of the circulation system. For example, since the number of sections is four in the embodiment illustrated in FIGS. 2 and 3, an embodiment in which the number of times of the step α is two is described, and since the number of sections is three in the embodiment illustrated in FIGS. 4 and 5, an embodiment in which the number of times of the step α is one is described.

Preferred embodiments of combinations of sub-steps in steps (A) and (B) will be described below. These embodiments can be performed by using the system of FIG. 1 or a system capable of carrying out a target embodiment conforming to the system of FIG. 1. Furthermore, the embodiments described below are merely examples of the present invention, and for example, from the viewpoint of relative desorption strength, two or more kinds of eluents positioned as an eluent d-I described below can be prepared, and the kind of the eluent d-I to be used can also be changed between sub-steps of supplying the eluent d-I. The same applies to the eluents d-II to d-IV.

That is, regarding the "eluent d-I" in an embodiment of the present invention, in the case of using the "eluent d-I" in different sub-steps in the embodiment, the "eluents d-I" used in different sub-steps may be the same as or different from each other. The same applies to the eluents d-II to d-IV.

### -Embodiment 1-

In Embodiment 1, a circulation system having four or more unit packed columns is used. Here, it is assumed that this circulation system is divided into four sections 1 to 4 that are annularly continuous from the upstream side to the downstream side such that each section has at least one of the unit packed column. Further, as the eluent, four kinds of eluents d-I to d-IV each having controlled desorption strength are used.

In this Embodiment 1, the following sub-steps (A1-1), (A2-1), (A3-1), and (A4-1) are performed in step (A).

### <Sub-step (A1-1)>

This sub-step includes supplying an eluent d-II from an eluent supply port D-II while using an upstream end of the section 1 as the eluent supply port D-II, extracting the intermediately adsorptive fraction from the extraction port B while using a downstream end of the section 2 as the extraction port B, supplying the feed solution from the feed solution supply port F while using an upstream end of the section 3 as the feed solution supply port F, and extracting the weakly adsorptive fraction from the extraction port A while using a downstream end of the section 4 as the extraction port A,
thereby most strengthening strongest desorption strength of the eluent passing through the sections 1 and 2, and weakening desorption strength of the eluent passing through the sections 3 and 4 more than the desorption strength of the eluent passing through the sections 1 and 2.

In the sub-step (A1-1), the supply of each liquid and the extraction of the weakly adsorptive fraction described above are continuously performed (that is, in the sub-step (A1-1), at all times, all of the supply of each liquid and the extraction of the weakly adsorptive fraction described above are performed without intermission). The same applies to each sub-step described hereinafter.

Furthermore, in the present invention and the present specification, the description of the strength level of desorption strength of the eluent described in each sub-step corresponds to the description of the strength level of desorption strength of the eluent in the relevant sub-step but does not correspond to the description of the strength level of desorption strength of the eluent in different sub-steps. For example, in a case where the desorption strength of the eluent passing through the section 1 is described as strongest in one sub-step constituting step (A) and the desorption strength of the eluent passing through the section 1 is described as strongest in another sub-step constituting step (A), the desorption strength of the eluent passing through the section 1 in the one sub-step and the desorption strength of the eluent passing through the section 1 in another sub-step may be the same as or different from each other.

### <Sub-step (A2-1)>

This sub-step includes supplying an eluent d-II from an eluent supply port D-II while using an upstream end of the section 1 as the eluent supply port D-II, extracting the intermediately adsorptive fraction from the extraction port B while using a downstream end of the section 2 as the extraction port B, supplying an eluent d-IV from an eluent supply port D-IV while using an upstream end of the section 3 as the eluent supply port D-IV, and extracting the weakly adsorptive fraction from the extraction port A while using a downstream end of the section 4 as the extraction port A,
thereby most strengthening strongest desorption strength of the eluent passing through the sections 1 and 2, and weakening desorption strength of the eluent passing through the sections 3 and 4 more than the desorption strength of the eluent passing through the sections 1 and 2.

The eluent supply port D-IV in this sub-step (A2-1) is provided on the same pipe as that for the feed solution supply port F in the sub-step (A1-1).

### <Sub-step (A3-1)>

This sub-step includes supplying an eluent d-I from an eluent supply port D-I while using an upstream end of the section 1 as the eluent supply port D-I, extracting the strongly adsorptive fraction from the extraction port C while using a downstream end of the section 1 as the extraction port C, supplying an eluent d-II from an eluent supply port D-II while using an upstream end of the section 2 as the eluent supply port D-II, supplying an eluent d-III from an eluent supply port D-III while using an upstream end of the section 3 as the eluent supply port D-III, supplying an eluent d-IV from an eluent supply port D-IV while using an upstream end of the section 4 as the eluent supply port D-IV, and extracting the weakly adsorptive fraction from the extraction port A while using a downstream end of the section 4 as the extraction port A,
thereby most strengthening strongest desorption strength of the eluent passing through the section 1, weakening desorption strength of the eluent passing through the section 2 more than the desorption strength of the eluent passing through the section 1, weakening desorption strength of the eluent passing through the section 3 more than the desorption strength of the eluent passing through the section 2, and weakening desorption strength of the eluent passing through the section 4 more than the desorption strength of the eluent passing through the section 3.

### <Sub-step (A4-1)>

This sub-step includes supplying an eluent d-II from an eluent supply port D-II while using an upstream end of the section 2 as the eluent supply port D-II, supplying an eluent d-III from an eluent supply port D-III while using an upstream end of the section 3 as the eluent supply port D-III, supplying an eluent d-IV from an eluent supply port D-IV while using an upstream end of the section 4 as the eluent supply port D-IV, and extracting the weakly adsorptive fraction from the extraction port A while using a downstream end of the section 1 as the extraction port A,
thereby most strengthening strongest desorption strength of the eluent passing through the section 2, weakening desorption strength of the eluent passing through the section 3 more than the desorption strength of the eluent passing through the section 2, weakening desorption strength of the eluent passing through the section 4 more than the desorption strength of the eluent passing through the section 3, and making the same desorption strength of the eluent passing through the section 1 as the desorption strength of the eluent passing through the section 4.

As an example of the sub-step (A1-1), a sub-step of performing the following sub-step (A1-1ex) is mentioned, but the sub-step (A1-1) is not limited to the sub-step (A1-1ex).

In the following example, it is described that the eluent d4 has a weaker desorption strength than the eluent d3, but the desorption strength of the eluent d3 may be the same as that of the eluent d4. The same applies to the following examples.

### <Sub-step (A1-1ex)>

The eluent d2 having the second strongest desorption strength of four kinds of eluents is supplied from an eluent supply port D2 while using an upstream end of the section 1 as the eluent supply port D2,
the intermediately adsorptive fraction is extracted from an intermediately adsorptive fraction extraction port B while using a downstream end of the section 2 as the intermediately adsorptive fraction extraction port B,
the feed solution is supplied from a feed solution supply port F while using an upstream end of the section 3 as the feed solution supply port F, and
the weakly adsorptive fraction is extracted from a weakly adsorptive fraction extraction port A while using a downstream end of the section 4 as the weakly adsorptive fraction extraction port A.

As an example of the sub-step (A2-1), a sub-step of performing the following sub-step (A2-1ex) is mentioned, but the sub-step (A2-1) is not limited to the sub-step (A2-1ex).

### <Sub-step (A2-1ex)>

The eluent d2 is supplied from the eluent supply port D2,
the intermediately adsorptive fraction is extracted from the intermediately adsorptive fraction extraction port B,
the eluent d4 having the weakest desorption strength of four kinds of eluents is supplied from an eluent supply port D4 while using an upstream end of the section 3 as the eluent supply port D4, and
the weakly adsorptive fraction is extracted from the weakly adsorptive fraction extraction port A.

The eluent supply port D4 in this sub-step (A2-1ex) is provided on the same pipe as that for the feed solution supply port F in the sub-step (A1-1ex). The eluent supply port D2 in this sub-step (A2-1ex) is the same as the eluent supply port D2 in the sub-step (A1-1ex).

As an example of the sub-step (A3-1), a sub-step of performing the following sub-step (A3-1ex) is mentioned, but the sub-step (A3-1) is not limited to the sub-step (A3-1ex).

### <Sub-step (A3-1ex)>

The eluent d1 having the strongest desorption strength of four kinds of eluents is supplied from an eluent supply port D1 while using an upstream end of the section 1 as the eluent supply port D1,
the strongly adsorptive fraction is extracted from a strongly adsorptive fraction extraction port C while using a downstream end of the section 1 as the strongly adsorptive fraction extraction port C,
the eluent d2 having the second strongest desorption strength of the four kinds of eluents is supplied from an eluent supply port D2 while using an upstream end of the section 2 as the eluent supply port D2,
the eluent d3 having the third strongest desorption strength of the four kinds of eluents is supplied from an eluent supply port D3 while using an upstream end of the section 3 as the eluent supply port D3,
the eluent d4 having the weakest desorption strength of the four kinds of eluents is supplied from an eluent supply port D4 while using an upstream end of the section 4 as the eluent supply port D4, and
the weakly adsorptive fraction is extracted from the weakly adsorptive fraction extraction port A.

The eluent supply port D1 in this sub-step (A3-1ex) is provided on the same pipe as that for the eluent supply port D2 in the sub-step (A2-1ex). The eluent supply port D3 in this sub-step (A3-1ex) is provided on the same pipe as that for the eluent supply port D4 in the sub-step (A2-1ex).

As an example of the sub-step (A4-1), a sub-step of performing the following sub-step (A4-1ex) is mentioned, but the sub-step (A4-1) is not limited to the sub-step (A4-1ex).

### <Sub-step (A4-1ex)>

The eluent d2 is supplied from the eluent supply port D2,
the eluent d3 is supplied from the eluent supply port D3,
the eluent d4 is supplied from the eluent supply port D4, and
the weakly adsorptive fraction is extracted from a weakly adsorptive fraction extraction port A while using a downstream end of the section 1 as the weakly adsorptive fraction extraction port A.

The eluent supply ports D2, D3, and D4 in this sub-step (A4-1ex) are the same as the eluent supply ports D2, D3, and D4 in the sub-step (A3-1ex).

FIG. 2 illustrates a flow diagram in a case where the sub-steps (A1-1ex), (A2-1ex), (A3-1ex), and (A4-1ex) are sequentially performed in step (A). In FIG. 2, a square enclosure indicates one section, and the number in the enclosure indicates the number of the section (the number is given in order from left).

In this Embodiment 1, in step (B), the following sub-steps (B1-1), (B2-1), and (B3-1) are provided as one set, and this set is performed twice. In this embodiment, each sub-step of the second time is denoted as (B1'-1), (B2'-1), and (B3'-1), respectively. That is, in step (B), the following sub-steps (B1-1) to (B3-1) and the following sub-steps (B1 '-1) to (B3'-1) are performed.

### <Sub-step (B1-1)>

This sub-step includes supplying an eluent d-II from an eluent supply port D-II while using an upstream end of the section 2 as the eluent supply port D-II, supplying an eluent d-III from an eluent supply port D-III while using an upstream end of the section 4 as the eluent supply port D-III, supplying an eluent d-IV from an eluent supply port D-IV while using an upstream end of the section 1 as the eluent supply port D-IV, and extracting the weakly adsorptive fraction from the extraction port A while using a downstream end of the section 1 as the extraction port A,
thereby most strengthening strongest desorption strength of the eluent passing through the sections 2 and 3, weakening desorption strength of the eluent passing through the section 4 more than the desorption strength of the eluent passing through the sections 2 and 3, and weakening desorption strength of the eluent passing through the section 1 more than the desorption strength of the eluent passing through the section 4.

### <Sub-step (B2-1)>

This sub-step includes supplying an eluent d-I from an eluent supply port D-I while using an upstream end of the section 2 as the eluent supply port D-I, extracting the strongly adsorptive fraction from the extraction port C while using a downstream end of the section 2 as the extraction port C, supplying an eluent d-II from an eluent supply port D-II while using an upstream end of the section 3 as the eluent supply port D-II, supplying an eluent d-III from an eluent supply port D-III while using an upstream end of the section 4 as the eluent supply port D-III, supplying an eluent d-IV from an eluent supply port D-IV while using an upstream end of the section 1 as the eluent supply port D-IV, and extracting the weakly adsorptive fraction from the extraction port A while using a downstream end of the section 1 as the extraction port A,
thereby most strengthening strongest desorption strength of the eluent passing through the section 2, weakening desorption strength of the eluent passing through the section 3 more than the desorption strength of the eluent passing through the section 2, weakening desorption strength of the eluent passing through the section 4 more than the desorption strength of the eluent passing through the section 3, and weakening desorption strength of the eluent passing through the section 1 more than the desorption strength of the eluent passing through the section 4.

### <Sub-step (B3-1)>

This sub-step includes supplying an eluent d-II from an eluent supply port D-II while using an upstream end of the section 3 as the eluent supply port D-II, supplying an eluent d-III from an eluent supply port D-III while using an upstream end of the section 4 as the eluent supply port D-III, supplying an eluent d-IV from an eluent supply port D-IV while using an upstream end of the section 1 as the eluent supply port D-IV, and extracting the weakly adsorptive fraction from the extraction port A while using a downstream end of the section 2 as the extraction port A,
thereby most strengthening strongest desorption strength of the eluent passing through the section 3, weakening desorption strength of the eluent passing through the section 4 more than the desorption strength of the eluent passing through the section 3, weakening desorption strength of the eluent passing through the section 1 more than the desorption strength of the eluent passing through the section 4, and making the same desorption strength of the eluent passing through the section 2 as the desorption strength of the eluent passing through the section 1.

### <Sub-step (B1'-1)>

This sub-step includes supplying an eluent d-II from an eluent supply port D-II while using an upstream end of the section 3 as the eluent supply port D-II, supplying an eluent d-III from an eluent supply port D-III while using an upstream end of the section 1 as the eluent supply port D-III, supplying an eluent d-IV from an eluent supply port D-IV while using an upstream end of the section 2 as the eluent supply port D-IV, and extracting the weakly adsorptive fraction from the extraction port A while using a downstream end of the section 2 as the extraction port A,
thereby most strengthening strongest desorption strength of the eluent passing through the sections 3 and 4, weakening desorption strength of the eluent passing through the section 1 more than the desorption strength of the eluent passing through the sections 3 and 4, and weakening desorption strength of the eluent passing through the section 2 more than the desorption strength of the eluent passing through the section 1.

### <Sub-step (B2'-1)>

This sub-step includes supplying an eluent d-I from an eluent supply port D-I while using an upstream end of the section 3 as the eluent supply port D-I, extracting the strongly adsorptive fraction from the extraction port C while using a downstream end of the section 3 as the extraction port C, supplying an eluent d-II from an eluent supply port D-II while using an upstream end of the section 4 as the eluent supply port D-II, supplying an eluent d-III from an eluent supply port D-III while using an upstream end of the section 1 as the eluent supply port D-III, supplying an eluent d-IV from an eluent supply port D-IV while using an upstream end of the section 2 as the eluent supply port D-IV, and extracting the weakly adsorptive fraction from the extraction port A while using a downstream end of the section 2 as the extraction port A,
thereby most strengthening strongest desorption strength of the eluent passing through the section 3, weakening desorption strength of the eluent passing through the section 4 more than the desorption strength of the eluent passing through the section 3, weakening desorption strength of the eluent passing through the section 1 more than the desorption strength of the eluent passing through the section 4, and weakening desorption strength of the eluent passing through the section 2 more than the desorption strength of the eluent passing through the section 1.

### <Sub-step (B3'-1)>

This sub-step includes supplying an eluent d-II from an eluent supply port D-II while using an upstream end of the section 4 as the eluent supply port D-II, supplying an eluent d-III from an eluent supply port D-III while using an upstream end of the section 1 as the eluent supply port D-III, supplying an eluent d-IV from an eluent supply port D-IV while using an upstream end of the section 2 as the eluent supply port D-IV, and extracting the weakly adsorptive fraction from the extraction port A while using a downstream end of the section 3 as the extraction port A,
thereby most strengthening strongest desorption strength of the eluent passing through the section 4, weakening desorption strength of the eluent passing through the section 1 more than the desorption strength of the eluent passing through the section 4, weakening desorption strength of the eluent passing through the section 2 more than the desorption strength of the eluent passing through the section 1, and making the same desorption strength of the eluent passing through the section 3 as the desorption strength of the eluent passing through the section 2.

As an example of the sub-step (B1-1), a sub-step of performing the following sub-step (B1-1ex) is mentioned, but the sub-step (B1-1) is not limited to the sub-step (B1-1ex).

### <Sub-step (B1-1ex)>

The eluent d2 having the second strongest desorption strength of the four kinds of eluents is supplied from an eluent supply port D2 while using an upstream end of the section 2 as the eluent supply port D2,
the eluent d3 having the third strongest desorption strength of the four kinds of eluents is supplied from an eluent supply port D3 while using an upstream end of the section 4 as the eluent supply port D3,
the eluent d4 having the weakest desorption strength of the four kinds of eluents is supplied from an eluent supply port D4 while using an upstream end of the section 1 as the eluent supply port D4, and
the weakly adsorptive fraction is extracted from a weakly adsorptive fraction extraction port A while using a downstream end of the section 1 as the extraction port A.

The eluent supply port D2 in this sub-step (B1-1ex) is the same as the eluent supply port D2 in the sub-step (A4-1ex). The eluent supply port D3 in this sub-step (B1-1ex) is provided on the same pipe as that for the eluent supply port D4 in the sub-step (A4-1ex).

As an example of the sub-step (B2-1), a sub-step of performing the following sub-step (B2-1ex) is mentioned, but the sub-step (B2-1) is not limited to the sub-step (B2-1ex).

### <Sub-step (B2-1ex)>

The eluent d1 having the strongest desorption strength of four kinds of eluents is supplied from an eluent supply port D1 while using an upstream end of the section 2 as the eluent supply port D1,
the strongly adsorptive fraction is extracted from a strongly adsorptive fraction extraction port C while using a downstream end of the section 2 as the strongly adsorptive fraction extraction port C,
the eluent d2 having the second strongest desorption strength of the four kinds of eluents is supplied from an eluent supply port D2 while using an upstream end of the section 3 as the eluent supply port D2,
the eluent d3 is supplied from the eluent supply port D3,
the eluent d4 is supplied from the eluent supply port D4, and
the weakly adsorptive fraction is extracted from the weakly adsorptive fraction extraction port A.

The eluent supply port D1 in this sub-step (B2-1ex) is provided on the same pipe as that for the eluent supply port D2 in the sub-step (B1-1ex). The eluent supply ports D3 and D4 in this sub-step (B2-1ex) are the same as the eluent supply ports D3 and D4 in the sub-step (B1-1ex).

As an example of the sub-step (B3-1), a sub-step of performing the following sub-step (B3-1ex) is mentioned, but the sub-step (B3-1) is not limited to the sub-step (B3-1 ex).

### <Sub-step (B3-1ex)>

The eluent d2 is supplied from the eluent supply port D2,
the eluent d3 is supplied from the eluent supply port D3,
the eluent d4 is supplied from the eluent supply port D4, and
the weakly adsorptive fraction is extracted from a weakly adsorptive fraction extraction port A while using a downstream end of the section 2 as the weakly adsorptive fraction extraction port A.

The eluent supply ports D2, D3, and D4 in this sub-step (B3-1ex) are the same as the eluent supply ports D2, D3, and D4 in the sub-step (B2-1ex).

As an example of the sub-step (B1'-1), a sub-step of performing the following sub-step (B1'-1ex) is mentioned, but the sub-step (B1'-1) is not limited to the sub-step (B1'-1ex).

### <Sub-step (B1'-1ex)>

The eluent d2 having the second strongest desorption strength of the four kinds of eluents is supplied from an eluent supply port D2 while using an upstream end of the section 3 as the eluent supply port D2,
the eluent d3 having the third strongest desorption strength of the four kinds of eluents is supplied from an eluent supply port D3 while using an upstream end of the section 1 as the eluent supply port D3,
the eluent d4 having the weakest desorption strength of the four kinds of eluents is supplied from an eluent supply port D4 while using an upstream end of the section 2 as the eluent supply port D4, and
the weakly adsorptive fraction is extracted from a weakly adsorptive fraction extraction port A while using a downstream end of the section 2 as the extraction port A.

The eluent supply port D2 in this sub-step (B1'-1ex) is the same as the eluent supply port D2 in the sub-step (B3-1ex). The eluent supply port D3 in this sub-step (B1'-1ex) is provided on the same pipe as that for the eluent supply port D4 in the sub-step (B3-1ex).

As an example of the sub-step (B2'-1), a sub-step of performing the following sub-step (B2'-1ex) is mentioned, but the sub-step (B2'-1) is not limited to the sub-step (B2'-1ex).

### <Sub-step (B2'-1ex)>

The eluent d1 having the strongest desorption strength of four kinds of eluents is supplied from an eluent supply port D1 while using an upstream end of the section 3 as the eluent supply port D1,
the strongly adsorptive fraction is extracted from a strongly adsorptive fraction extraction port C while using a downstream end of the section 3 as the strongly adsorptive fraction extraction port C,
the eluent d2 having the second strongest desorption strength of the four kinds of eluents is supplied from an eluent supply port D2 while using an upstream end of the section 4 as the eluent supply port D2,
the eluent d3 is supplied from the eluent supply port D3,
the eluent d4 is supplied from the eluent supply port D4, and
the weakly adsorptive fraction is extracted from the weakly adsorptive fraction extraction port A.

The eluent supply port D1 in this sub-step (B2'-1ex) is provided on the same pipe as that for the eluent supply port D2 in the sub-step (B1'-1 ex). The eluent supply ports D3 and D4 in this sub-step (B2'-1ex) are the same as the eluent supply ports D3 and D4 in the sub-step (B1'-1ex).

As an example of the sub-step (B3'-1), a sub-step of performing the following sub-step (B3'-1ex) is mentioned, but the sub-step (B3'-1) is not limited to the sub-step (B3'-1ex).

### <Sub-step (B3'-1ex)>

The eluent d2 is supplied from the eluent supply port D2,
the eluent d3 is supplied from the eluent supply port D3,
the eluent d4 is supplied from the eluent supply port D4, and
the weakly adsorptive fraction is extracted from a weakly adsorptive fraction extraction port A while using a downstream end of the section 3 as the weakly adsorptive fraction extraction port A.

The eluent supply ports D2, D3, and D4 in this sub-step (B3'-1ex) are the same as the eluent supply ports D2, D3, and D4 in the sub-step (B2'-1ex).

FIG. 2 illustrates a flow diagram in a case where the sub-steps (B1-1ex), (B2-1ex), (B3-1ex), (B1'-1ex), (B2'-1ex), and (B3'-1ex) are sequentially performed in step (B). In FIG. 2, a square enclosure indicates one section, and the number in the enclosure indicates the number of the section (the number is given in order from left).

After step (B) in which the sub-steps (B1-1ex), (B2-1ex), (B3-1ex), (B1'-1ex), (B2'-1ex), and (B3'-1ex) are sequentially performed, the position of the weakly adsorptive fraction extraction port A in the sub-step (B3'-1ex) is kept as it is and the position of the weakly adsorptive fraction extraction port A is used as a reference while maintaining the relative positional relationship between the respective positions to construct the eluent supply port D2, the intermediately adsorptive fraction extraction port B, and the feed solution supply port F. The sub-step (A1-1ex) of the next cycle is then performed. FIG. 3 shows a flow diagram of steps (A) and (B) in the next cycle. As compared with the cycle shown in FIG. 2, in the next cycle shown in FIG. 3, all the supply ports and the extraction ports are shifted to the upstream side by one section (downstream side by three sections) while maintaining the relative positional relationship.

### -Embodiment 2-

In Embodiment 2, a circulation system having three or more unit packed columns is used. Here, it is assumed that this circulation system is divided into three sections 1 to 3 that are annularly continuous from the upstream side to the downstream side such that each section has at least one of the unit packed column. Further, as the eluent, four kinds of eluents d-I to d-IV each having controlled desorption strength are used.

In this Embodiment 2, the following sub-steps (A1-2), (A2-2), (A3-2), and (A4-2) are sequentially performed as step (A).

### <Sub-step (A1-2)>

This sub-step includes supplying an eluent d-II from an eluent supply port D-II while using an upstream end of the section 1 as the eluent supply port D-II, extracting the intermediately adsorptive fraction from the extraction port B while using a downstream end of the section 2 as the extraction port B, supplying the feed solution from the feed solution supply port F while using an upstream end of the section 3 as the feed solution supply port F, and extracting the weakly adsorptive fraction from the extraction port A while using a downstream end of the section 3 as the extraction port A,
thereby most strengthening strongest desorption strength of the eluent passing through the sections 1 and 2, and weakening desorption strength of the eluent passing through the section 3 more than the desorption strength of the eluent passing through the sections 1 and 2.

### <Sub-step (A2-2)>

This sub-step includes supplying an eluent d-II from an eluent supply port D-II while using an upstream end of the section 1 as the eluent supply port D-II, extracting the intermediately adsorptive fraction from the extraction port B while using a downstream end of the section 2 as the extraction port B, supplying an eluent d-IV from an eluent supply port D-IV while using an upstream end of the section 3 as the eluent supply port D-IV, and extracting the weakly adsorptive fraction from the extraction port A while using a downstream end of the section 3 as the extraction port A,
thereby most strengthening strongest desorption strength of the eluent passing through the sections 1 and 2, and weakening desorption strength of the eluent passing through the section 3 more than the desorption strength of the eluent passing through the sections 1 and 2.

The eluent supply port D-IV in this sub-step (A2-2) is provided on the same pipe as that for the feed solution supply port F in the sub-step (A1-2).

### <Sub-step (A3-2)>

This sub-step includes supplying an eluent d-I from an eluent supply port D-I while using an upstream end of the section 1 as the eluent supply port D-I, extracting the strongly adsorptive fraction from the extraction port C while using a downstream end of the section 1 as the extraction port C, supplying an eluent d-II from an eluent supply port D-II while using an upstream end of the section 2 as the eluent supply port D-II, supplying an eluent d-III from an eluent supply port D-III while using an upstream end of the section 3 as the eluent supply port D-III, and extracting the weakly adsorptive fraction from the extraction port A while using a downstream end of the section 3 as the extraction port A,
thereby most strengthening strongest desorption strength of the eluent passing through the section 1, weakening desorption strength of the eluent passing through the section 2 more than the desorption strength of the eluent passing through the section 1, and weakening desorption strength of the eluent passing through the section 3 more than the desorption strength of the eluent passing through the section 2.

### <Sub-step (A4-2)>

This sub-step includes supplying an eluent d-II from an eluent supply port D-II while using an upstream end of the section 2 as the eluent supply port D-II, supplying an eluent d-III from an eluent supply port D-III while using an upstream end of the section 3 as the eluent supply port D-III, supplying an eluent d-IV from an eluent supply port D-IV while using an upstream end of the section 1 as the eluent supply port D-IV, and extracting the weakly adsorptive fraction from the extraction port A while using a downstream end of the section 1 as the extraction port A,
thereby most strengthening strongest desorption strength of the eluent passing through the section 2, weakening desorption strength of the eluent passing through the section 3 more than the desorption strength of the eluent passing through the section 2, and weakening desorption strength of the eluent passing through the section 1 more than the desorption strength of the eluent passing through the section 3.

As an example of the sub-step (A1-2), a sub-step of performing the following sub-step (A1-2ex) is mentioned, but the sub-step (A1-2) is not limited to the sub-step (A1-2ex).

In the following example, it is described that the eluent d4 has a weaker desorption strength than the eluent d3, but the desorption strength of the eluent d3 may be the same as that of the eluent d4. The same applies to the following examples.

### <Sub-step (A1-2ex)>

The eluent d2 having the second strongest desorption strength of four kinds of eluents is supplied from an eluent supply port D1 while using an upstream end of the section 1 as the eluent supply port D2, the intermediately adsorptive fraction is extracted from an intermediately adsorptive fraction extraction port B while using a downstream end of the section 2 as the intermediately adsorptive fraction extraction port B, the feed solution is supplied from a feed solution supply port F while using an upstream end of the section 3 as the feed solution supply port F, and the weakly adsorptive fraction is extracted from a weakly adsorptive fraction extraction port A while using a downstream end of the section 3 as the weakly adsorptive fraction extraction port A.

As an example of the sub-step (A2-2), a sub-step of performing the following sub-step (A2-2ex) is mentioned, but the sub-step (A2-2) is not limited to the sub-step (A2-2ex).

### <Sub-step (A2-2ex)>

The eluent d2 is supplied from the eluent supply port D2,
the intermediately adsorptive fraction is extracted from the intermediately adsorptive fraction extraction port B,
the eluent d4 having the weakest desorption strength of four kinds of eluents is supplied from an eluent supply port D4 while using an upstream end of the section 3 as the eluent supply port D4, and
the weakly adsorptive fraction is extracted from the weakly adsorptive fraction extraction port A.

The eluent supply port D4 in this sub-step (A2-2ex) is provided on the same pipe as that for the feed solution supply port F in the sub-step (A1-2ex). The eluent supply port D2 in this sub-step (A2-2ex) is the same as the eluent supply port D2 in the sub-step (A1-2ex).

As an example of the sub-step (A3-2), a sub-step of performing the following sub-step (A3-2ex) is mentioned, but the sub-step (A3-2) is not limited to the sub-step (A3-2ex).

### <Sub-step (A3-2ex)>

The eluent d1 having the strongest desorption strength of four kinds of eluents is supplied from an eluent supply port D1 while using an upstream end of the section 1 as the eluent supply port D1,
the strongly adsorptive fraction is extracted from a strongly adsorptive fraction extraction port C while using a downstream end of the section 1 as the strongly adsorptive fraction extraction port C,
the eluent d2 having the second strongest desorption strength of the four kinds of eluents is supplied from an eluent supply port D2 while using an upstream end of the section 2 as the eluent supply port D2,
the eluent d3 having the third strongest desorption strength of the four kinds of eluents is supplied from an eluent supply port D3 while using an upstream end of the section 3 as the eluent supply port D3, and
the weakly adsorptive fraction is extracted from the weakly adsorptive fraction extraction port A.

The eluent supply port D1 in this sub-step (A3-2ex) is provided on the same pipe as that for the eluent supply port D2 in the sub-step (A2-2ex). The eluent supply port D3 in this sub-step (A3-2ex) is provided on the same pipe as that for the eluent supply port D4 in the sub-step (A2-2ex).

As an example of the sub-step (A4-2), a sub-step of performing the following sub-step (A4-2ex) is mentioned, but the sub-step (A4-2) is not limited to the sub-step (A4-2ex).

### <Sub-step (A4-2ex)>

The eluent d2 is supplied from the eluent supply port D2,
the eluent d3 is supplied from the eluent supply port D3,
the eluent d4 having the weakest desorption strength of the four kinds of eluents is supplied from an eluent supply port D4 while using an upstream end of the section 1 as the eluent supply port D4, and
the weakly adsorptive fraction is extracted from a weakly adsorptive fraction extraction port A while using a downstream end of the section 1 as the extraction port A.

The eluent supply ports D2 and D3 in this sub-step (A4-2ex) are the same as the eluent supply ports D2 and D3 in the sub-step (A3-2ex).

FIG. 4 illustrates a flow diagram in a case where the sub-steps (A1-2ex), (A2-2ex), (A3-2ex), and (A4-2ex) are sequentially performed in step (A). In FIG. 4, a square enclosure indicates one section, and the number in the enclosure indicates the number of the section (the number is given in order from left).

In this Embodiment 2, the following sub-steps (B1-2) and (B2-2) are performed in step (B).

### <Sub-step (B1-2)>

This sub-step includes supplying an eluent d-I from an eluent supply port D-I while using an upstream end of the section 2 as the eluent supply port D-I, extracting the strongly adsorptive fraction from the extraction port C while using a downstream end of the section 2 as the extraction port C, supplying an eluent d-II from an eluent supply port D-II while using an upstream end of the section 3 as the eluent supply port D-II, supplying an eluent d-III from an eluent supply port D-III while using an upstream end of the section 1 as the eluent supply port D-III, and extracting the weakly adsorptive fraction from the extraction port A while using a downstream end of the section 1 as the extraction port A,
thereby most strengthening strongest desorption strength of the eluent passing through the section 2, weakening desorption strength of the eluent passing through the section 3 more than the desorption strength of the eluent passing through the section 2, and weakening desorption strength of the eluent passing through the section 1 more than the desorption strength of the eluent passing through the section 3.

### <Sub-step (B2-2)>

This sub-step includes supplying an eluent d-II from an eluent supply port D-II while using an upstream end of the section 3 as the eluent supply port D-II, supplying an eluent d-III from an eluent supply port D-III while using an upstream end of the section 1 as the eluent supply port D-III, and extracting the weakly adsorptive fraction from the extraction port A while using a downstream end of the section 2 as the extraction port A,
thereby most strengthening strongest desorption strength of the eluent passing through the section 3, weakening desorption strength of the eluent passing through the section 1 more than the desorption strength of the eluent passing through the section 3, and weakening desorption strength of the eluent passing through the section 2 more than the desorption strength of the eluent passing through the section 1.

As an example of the sub-step (B1-2), a sub-step of performing the following sub-step (B1-2ex) is mentioned, but the sub-step (B1-2) is not limited to the sub-step (B1-2ex).

### <Sub-step (B1-2ex)>

The eluent d1 having the strongest desorption strength of four kinds of eluents is supplied from an eluent supply port D1 while using an upstream end of the section 2 as the eluent supply port D1,
the strongly adsorptive fraction is extracted from a strongly adsorptive fraction extraction port C while using a downstream end of the section 2 as the strongly adsorptive fraction extraction port C,
the eluent d2 having the second strongest desorption strength of the four kinds of eluents is supplied from an eluent supply port D2 while using an upstream end of the section 3 as the eluent supply port D2,
the eluent d3 having the third strongest desorption strength of the four kinds of eluents is supplied from an eluent supply port D3 while using an upstream end of the section 1 as the eluent supply port D3, and
the weakly adsorptive fraction is extracted from a weakly adsorptive fraction extraction port A while using a downstream end of the section 1 as the weakly adsorptive fraction extraction port A.

The eluent supply ports D1, D2, and D3 in this sub-step (B1-2ex) are provided on the same pipes as those for the eluent supply ports D2, D3, and D4 in the sub-step (A4-2ex).

As an example of the sub-step (B2-2), a sub-step of performing the following sub-step (B2-2ex) is mentioned, but the sub-step (B2-2) is not limited to the sub-step (B2-2ex).

### <Sub-step (B2-2ex)>

The eluent d2 is supplied from the eluent supply port D2,
the eluent d3 is supplied from the eluent supply port D3,
the eluent d4 having the weakest desorption strength of the four kinds of eluents is supplied from an eluent supply port D4 while using an upstream end of the section 2 as the eluent supply port D4, and
the weakly adsorptive fraction is extracted from a weakly adsorptive fraction extraction port A while using a downstream end of the section 2 as the weakly adsorptive fraction extraction port A.

The eluent supply ports D2 and D3 in this sub-step (B2-2ex) are the same as the eluent supply ports D2 and D3 in the sub-step (B1-2ex).

FIG. 4 illustrates a flow diagram in a case where the sub-steps (B1-2ex) and (B2-2ex) are sequentially performed in step (B). In FIG. 4, a square enclosure indicates one section, and the number in the enclosure indicates the number of the section (the number is given in order from left).

After step (B) in which the sub-steps (B1-2ex) and (B2-2ex) are sequentially performed, the position of the weakly adsorptive fraction extraction port A in the sub-step (B2-2ex) is kept as it is and the position of the weakly adsorptive fraction extraction port A is used as a reference while maintaining the relative positional relationship between the respective positions to construct the eluent supply port D2, the intermediately adsorptive fraction extraction port B, and the feed solution supply port F. The sub-step (A1-2ex) of the next cycle is then performed. FIG. 5 shows a flow diagram of steps (A) and (B) in the next cycle. As compared with the cycle shown in FIG. 4, in the next cycle shown in FIG. 5, all the supply ports and the extraction ports are shifted to the upstream side by one section (downstream side by two sections) while maintaining the relative positional relationship.

In the method of the present invention, supply of a target liquid to a target place or extraction of a target liquid from a target extraction place can be performed by appropriately adjusting the operation of a pump at each place and opening/closing of a valve at each place which are provided in a circulation system. That is, methods of supplying a target fluid and extracting a target fraction in a circulation system are publicly known. Furthermore, the supply flow rate and the extraction flow rate of each liquid can also be appropriately set depending on purposes such as processing efficiency.

In the method of the present invention, a purification target component may be any one of a strongly adsorptive component, an intermediately adsorptive component, and a weakly adsorptive component, and in particular, the method of the present invention is a suitable method for purifying an intermediately adsorptive component. The method of the present invention can be suitably used for, for example, purification of protein. Since an intermediately adsorptive component can be obtained at a high purity by the method of the present invention, the method of the present invention is a suitable method for obtaining the target protein from a feed solution containing a target protein as well as a digest or aggregate thereof.

The protein is not particularly limited, and for example, a biopolymer, such as an antibody, a peptide, nucleic acid, and a fusion protein, can be used as a purification target component. The method of the present invention is preferably used for purification of an antibody. The term "antibody" refers to an intact antibody or a binding fragment thereof that competes with an intact antibody for specific binding. Examples of the binding fragment include, but are not limited to, F(ab), F(ab'), F(ab')₂, Fv, or a single chain antibody.

In the present invention, the "antibody" may be a naturally occurring antibody, a chimeric antibody, and an antibody fragmented by an enzyme or the like (for example, F(ab')₂ fragment, Fab' fragment, or Fab fragment). Also included are single chain antibodies, dimers (diabodies) or trimers (triabodies) thereof, or minibodies. Alternatively, the antibody may be a single domain antibody. Incidentally, these are merely examples, all of proteins having a specific binding ability with respect to antigens and derivatives thereof are included in the concept of the antibody in the present invention.

An antibody highly purified by the method of the present invention can be applied as an antibody drug. That is, an antibody contained in a feed solution is fractionated by applying the method of the present invention, so that a method of producing an antibody drug can be provided. More specifically, according to the method of the present invention, an antibody drug can be obtained by using a culture medium for antibody-producing cells and/or an extract from antibody-producing cells as a feed solution and fractionating an antibody contained in the feed solution. In the present invention, the meaning of the term "culture medium for antibody-producing cells" or "extract from antibody-producing cells" includes a state where the culture medium for antibody-producing cells or the extract from antibody-producing cells is subjected to various processing such as centrifugation and/or chromatography to be fractionated, purified, etc. to some extent.

In the method of the present invention, an adsorbent to be packed in the unit packed column is appropriately selected according to a purification target component, and various adsorbents can be adopted. For example, a strongly acidic cation-exchange resin, a weakly acidic cation-exchange resin, a strongly basic anion-exchange resin, a weakly basic anion-exchange resin, a synthetic adsorbent, zeolite, silica gel, functional group-modified silica gel (preferably octadecylsilyl-modified silica gel), other materials for gel filtration chromatography, or an affinity adsorbent can be used as the adsorbent.

In a case where a purification target component is protein, the adsorbent is preferably an ion-exchange resin. In particular, a cation-exchange resin can be suitably used.

The simulated moving-bed type chromatographic separation system of the present invention is a system for implementing the method of the present invention. That is, the simulated moving-bed type chromatographic separation system of the present invention is a system having the configuration of the circulation system described above, in which the circulation system can sequentially repeat the operation of the first process and the operation of the second process, or the operation of step (A) and the operation of step (B) as described above.

### EXAMPLES

Hereinafter, the present invention will be described in more detail on the basis of Examples; however, the present invention is not limited to the following Examples.

### [Preparation of feed solution]

Cells producing human immune globulin G1 (IgG1) were cultured, the pH and conductivity of the supernatant of the culture medium was adjusted to prepare a feed solution. The contents of the antibody and impurities contained in this supernatant are as described in Table 1 below. In the following Table, the antibody was a protein contained in a fraction with a peak molecular weight at 150,000, i.e., a molecular weight of 50,000 or higher and less than 300,000; LMW was a fraction with a molecular weight of less than 50,000; and HMW was a fraction with a molecular weight of 300,000 or higher. Further, among the LMW, a fraction having a high molecular weight was designated as LMW-1, and a fraction having a low molecular weight was designated as LMW-2. Likewise, among the HMW, a fraction having a high molecular weight was designated as HMW-1, and a fraction having a low molecular weight was designated as HMW-2. The component compositions described below were determined by high-performance liquid chromatography (HPLC) using an analytical column (Tosoh Corporation TSKgel G3000SWXL).

**Table 1**

| Component in supernatant of culture medium | Purity (%) |
|---|---|
| HMW-1 | 0.05 |
| H M W-2 | 0.93 |
| Antibody | 88.4 |
| LMW-1 | 0.00 |
| LMW-2 | 10.62 |

### [Adsorbent used in unit packed column (column)]

A cation exchange resin (trade name: POROS (registered trademark) XS Resin, manufactured by Thermo Fisher Scientific) was used as an adsorbent.

### [Eluent]

Eluents d1 to d4 were prepared using the following liquids A and B so as to have conductivities described in the following Table 2. Note that the conductivity of the feed solution (F) was 5.0 mS/cm, and the pH was 6.0.

### <A liquid>

50 mM MES buffer pH 6.0

### <B liquid>

50 mM MES buffer containing NaCl at a concentration of 2.0 M (117 g/L)

### (pH 6.0)

**Table 2**

| No. | Eluent type | pH | Conductivity (mS/cm) |
|---|---|---|---|
| d1 | MES Buffer +NaCl | 6.0 | 128 |
| d2 | MES Buffer +NaCl | 6.0 | 21.0 |
| d3 | MES Buffer +NaCl | 6.0 | 5.0 |
| d4 | MES Buffer +NaCl | 6.0 | 5.0 |

### -Example 1-

### <Operation conditions>

The following steps 1 to 10 shown in Table 3 below were sequentially performed. The flow diagram for the steps 1 to 10 is shown in FIG. 2. In FIG. 2, the following steps 1 to 10 correspond to sub-steps (A1-1) to (B3'-1), respectively. In Table 3 below, the feed solution (F) was supplied from the feed solution supply port F, and the eluents d1 to d4 were supplied from the eluent supply ports D1 to D4, respectively.

### [Table 3]

**Table 3**

| Step | Time (sec) | Flow rate (mL/min.) | | | | |
|---|---|---|---|---|---|---|
| | | F | D1 | D2 | D3 | D4 |
| 1 | 475 | 0.50 | - | 1.00 | - | - |
| 2 | 65 | - | - | 1.00 | - | 1.38 |
| 3 | 60 | - | 2.00 | 1.00 | 0.20 | 1.00 |
| 4 | 250 | - | - | 1.00 | 0.20 | 1.00 |
| 5 | 150 | - | - | 1.00 | 0.20 | 1.00 |
| 6 | 60 | - | 2.00 | 1.00 | 0.20 | 1.00 |
| 7 | 240 | - | - | 1.00 | 0.20 | 1.00 |
| 8 | 150 | - | - | 1.00 | 0.20 | 1.00 |
| 9 | 60 | - | 2.00 | 1.00 | 0.20 | 1.00 |
| 10 | 240 | - | - | 1.00 | 0.20 | 1.00 |

### <Results>

### -Recovery percentage-

The antibody concentration, antibody amount, purity, and recovery percentage of the antibody in the intermediately adsorptive fraction recovered in the fifth cycle are shown in Table 4 below. A calibration curve for each antibody amount was prepared by high performance liquid chromatography (HPLC) using an analytical column (trade name: TSKgel G 3000 SWXL, manufactured by Tosoh Corporation), and the antibody concentration was calculated from the antibody area value obtained by HPLC. In addition, the amount of antibody was calculated by the product of the antibody concentration obtained by the above calculation and the amount of recovered liquid. This recovery percentage was calculated by 100 × [Antibody amount in the fraction]/[Antibody amount in the feed solution]. The purity of the antibody was calculated from [the antibody area value measured by high performance liquid chromatography (HPLC) using an analytical column (trade name: TSKgel G 3000 SWXL, manufactured by Tosoh Corporation)]/[the area value of all the components].

**Table 4**

| | Intermediately adsorptive fraction |
|---|---|
| Antibody concentration (mg/mL) | 2.693 |
| Antibody amount (mg) | 25.042 |
| Purity (%) | 98.72 |
| Recovery percentage (%) | 96.04 |

Table 4 has clearly demonstrated that the antibody purified to high purity can be recovered efficiently.

### -Example 2-

A combination of the sub-steps shown in FIG. 4 was adopted, and the feed solution was subjected to simulated moving-bed type chromatographic separation. Specifically, the number of unit packed columns was 3, and the above steps 1 to 4, 6, and 7 (provided that in steps 3 and 6, the eluent d4 was not supplied from the eluent supply port D4) were sequentially performed under the conditions. Note that steps 1 to 4, 6, and 7 (provided that the eluent d4 is not supplied in steps 3 and 6) correspond to sub-steps (A1-2) to (B2-2) illustrated in FIG. 4, respectively. The results of performing chromatographic separation by adjusting (optimizing) the flow rate and the supply time of each liquid have demonstrated that even in this embodiment, the antibody purified to high purity (purity of 98% or higher) can be recovered efficiently (recovery percentage of 96% or higher) as in Example 1.

The present invention has been described as related to the present embodiments. It is our intention that the present invention not be limited by any of the details of the description unless otherwise specified, but rather be construed broadly within its spirit and scope as set out in the attached claims.

The present application claims priority of Patent Application No. 2022-045536 filed in Japan on March 22, 2022, which is herein incorporated by reference as part of the present specification.

### DESCRIPTION OF SYMBOLS

- 100: Circulation system
- 10a, 10b, 10c, 10d: Unit packed column (column)
- Ab: Adsorbent
- R1, R2, R3, R4: Shutoff valve
- 2a, 2b, 2c, 2d: Weakly adsorptive fraction extracting line
- A1, A2, A3, A4: Weakly adsorptive fraction extracting valve
- 3a, 3b, 3c, 3d: Intermediately adsorptive fraction extracting line
- B1, B2, B3, B4: Intermediately adsorptive fraction extracting valve
- 4a, 4b, 4c, 4d: Strongly adsorptive fraction extracting line
- C1, C2, C3, C4: Strongly adsorptive fraction extracting valve
- T1, T2, T3, T4: Check valve
- 1: Pipe
- 2J: Weakly adsorptive fraction joining pipe
- 3J: Intermediately adsorptive fraction joining pipe
- 4J: Strongly adsorptive fraction joining pipe
- 6: Feed solution tank
- 7: Feed solution
- 8a, 8b, 8c, 8d: Eluent tank
- 9a, 9b, 9c, 9d: Eluent
- 11: Feed solution supplying line
- 11a, 11b, 11c, 11d: Divergent feed solution supplying line
- F1, F2, F3, F4: Feed solution supplying valve
- 12, 13, 14, 15: Eluent supplying line
- 12a, 12b, 12c, 12d: Divergent eluent supplying line
- 13a, 13b, 13c, 13d: Divergent eluent supplying line
- 14a, 14b, 14c, 14d: Divergent eluent supplying line
- 15a, 15b, 15c, 15d: Divergent eluent supplying line
- E1a, E2a, E3a, E4a: Eluent supplying valve
- E1b, E2b, E3b, E4b: Eluent supplying valve
- E1c, E2c, E3c, E4c: Eluent supplying valve
- E1d, E2d, E3d, E4d: Eluent supplying valve
- P1: Circulation pump
- P2: Feed solution supply pump
- P3, P4, P5, P6: Eluent supply pump

## Claims

1. A simulated moving-bed type chromatographic separation method comprising: using a circulation system in which three or more unit packed columns each filled with an adsorbent are connected in series and in an endless form via pipes, dividing the circulation system into at least three sections annularly continuous from an upstream side to a downstream side so that each section has at least one of the unit packed column, and separating, by using an eluent, a weakly adsorptive component, a strongly adsorptive component, and an intermediately adsorptive component having adsorption performance on the adsorbent, which performance is intermediate between the previous two components, the weakly adsorptive component, strongly adsorptive component, and intermediately adsorptive component being included in a feed solution, wherein a cycle containing a first process and a second process below is repeated:
<first process>
a process containing blocking circulation of the circulation system by setting a blocked position to a position upstream of the weakly adsorptive component-rich section, supplying the feed solution to a section downstream of the blocked position, and extracting, from the circulation system, the intermediately adsorptive component-rich intermediately adsorptive fraction from a section upstream of the blocked position; and
<second process>
a process containing extracting the strongly adsorptive component-rich strongly adsorptive fraction by controlling supply of the eluent and blocking of the circulation system without supplying the feed solution, and shifting a supply position of the eluent relative to the circulation system and an extraction position of the strongly adsorptive fraction to a downstream side in accordance with movement of the strongly adsorptive fraction,
wherein, in the cycle containing the first process and the second process, the extraction position of the strongly adsorptive fraction is shifted to a downstream side by 2 sections or more and the [number of sections] shifted toward the downstream side and the total number of sections are not the same.

2. A simulated moving-bed type chromatographic separation method comprising: using a circulation system in which three or more unit packed columns each filled with an adsorbent are connected in series and in an endless form via pipes, dividing the circulation system into at least three sections annularly continuous from an upstream side to a downstream side so that each section has at least one of the unit packed column, and separating, by using an eluent, a weakly adsorptive component, a strongly adsorptive component, and an intermediately adsorptive component having adsorption performance on the adsorbent, which performance is intermediate between the previous two components, the weakly adsorptive component, strongly adsorptive component, and intermediately adsorptive component being included in a feed solution, wherein a cycle containing step (A) and step (B) below is repeated:
<step (A)>
a step comprising:
sub-step (A1) of blocking circulation of the circulation system by setting a blocked position to a position upstream of the weakly adsorptive component-rich section, supplying the feed solution at a section downstream of the blocked position and extracting, from the circulation system, the weakly adsorptive component-rich weakly adsorptive fraction at a position further downstream of the blocked position, and supplying an eluent for desorbing the intermediately adsorptive component at a section upstream of the blocked position and extracting, from the circulation system, the intermediately adsorptive component-rich intermediately adsorptive fraction at a position downstream thereof,
sub-step (A2) of supplying an eluent for desorbing the weakly adsorptive component instead of supplying the feed solution and continuing extraction of the weakly adsorptive fraction and extraction of the intermediately adsorptive fraction,
sub-step (A3) of shifting the blocked position to an upstream side and supplying an eluent for desorbing the strongly adsorptive component at a position upstream of the blocked position having been shifted and extracting the strongly adsorptive component-rich strongly adsorptive fraction at a position further downstream thereof while continuing extraction of the weakly adsorptive fraction, and
sub-step (A4) of shifting the extraction position of the weakly adsorptive fraction to a downstream side and extracting the weakly adsorptive fraction while extracting neither the intermediately adsorptive fraction nor the strongly adsorptive fraction,
wherein, in step (A), the sub-steps (A1), (A2), (A3) and (A4) are performed in this sequence; and
<step (B)>
a step comprising step α of separately extracting each of the weakly adsorptive fraction or strongly adsorptive fraction not extracted in step (A) by controlling supply of the eluent and the blocked position without supplying the feed solution while keeping the extraction position of the weakly adsorptive fraction as it is, and then shifting the extraction position of the weakly adsorptive fraction to a downstream side and extracting only the weakly adsorptive fraction, wherein, in step (B), at least one of the step α is performed.

3. The simulated moving-bed type chromatographic separation method according to claim 2,
wherein the pipes of the circulation system are provided with a feed solution supply port F, an eluent supply port D, a weakly adsorptive fraction extraction port A, an intermediately adsorptive fraction extraction port B, and a strongly adsorptive fraction extraction port C,
wherein, in step (A), the feed solution supply port F, the extraction port A, the extraction port B, and the extraction port C are positioned in accordance with the following (a) to (c):
(a) the extraction port A is provided downstream of the feed solution supply port F with at least one section interposed therebetween;
(b) the extraction port B is provided on the pipe having the feed solution supply port F; and
(c) the extraction port C is provided upstream of the extraction port B with at least one section interposed therebetween; and
wherein, in step (B), the extraction port A and the extraction port C are positioned in accordance with the following (d):
(d) the extraction port A is provided downstream of the extraction port C with at least two sections interposed therebetween.

4. The simulated moving-bed type chromatographic separation method according to claim 2 or 3, wherein the number of times of step α performed in step (B) is [the number of sections -2] or [the number of sections] or more.

5. The simulated moving-bed type chromatographic separation method according to any one of claims 2 to 4, comprising: using a circulation system in which four or more unit packed columns each filled with an adsorbent are connected in series and in an endless form via pipes, and dividing the circulation system into four sections annularly continuous from an upstream side to a downstream side so that each section has at least one of the unit packed column, wherein sub-steps (A1) to (A4) in step (A) are sub-steps (A1-1) to (A4-1) below, and
wherein, in step (B), the following sub-steps (B1-1), (B2-1) and (B3-1) and sub-steps (B1'-1) (B2'-1) and (B3'-1) are performed in sequence:
<sub-step (A1-1)>
supplying an eluent d-II from an eluent supply port D-II while using an upstream end of the section 1 as the eluent supply port D-II, extracting the intermediately adsorptive fraction from an intermediately adsorptive fraction extraction port B while using a downstream end of the section 2 as the extraction port B, supplying the feed solution from a feed solution supply port F while using an upstream end of the section 3 as the feed solution supply port F, and extracting the weakly adsorptive fraction from a weakly adsorptive fraction extraction port A while using a downstream end of the section 4 as the extraction port A,
thereby most strengthening strongest desorption strength of the eluent passing through the sections 1 and 2, and weakening desorption strength of the eluent passing through the sections 3 and 4 more than the desorption strength of the eluent passing through the sections 1 and 2;
<sub-step (A2-1)>
supplying an eluent d-II from an eluent supply port D-II while using an upstream end of the section 1 as the eluent supply port D-II, extracting the intermediately adsorptive fraction from an intermediately adsorptive fraction extraction port B while using a downstream end of the section 2 as the extraction port B, supplying an eluent d-IV from an eluent supply port D-IV while using an upstream end of the section 3 as the eluent supply port D-IV, and extracting the weakly adsorptive fraction from a weakly adsorptive fraction extraction port A while using a downstream end of the section 4 as the extraction port A,
thereby most strengthening strongest desorption strength of the eluent passing through the sections 1 and 2, and weakening desorption strength of the eluent passing through the sections 3 and 4 more than the desorption strength of the eluent passing through the sections 1 and 2;
<sub-step (A3-1)>
supplying an eluent d-I from an eluent supply port D-I while using an upstream end of the section 1 as the eluent supply port D-I, extracting the strongly adsorptive fraction from a strongly adsorptive fraction extraction port C while using a downstream end of the section 1 as the extraction port C, supplying an eluent d-II from an eluent supply port D-II while using an upstream end of the section 2 as the eluent supply port D-II, supplying an eluent d-III from an eluent supply port D-III while using an upstream end of the section 3 as the eluent supply port D-III, supplying an eluent d-IV from an eluent supply port D-IV while using an upstream end of the section 4 as the eluent supply port D-IV, and extracting the weakly adsorptive fraction from a weakly adsorptive fraction extraction port A while using a downstream end of the section 4 as the extraction port A,
thereby most strengthening strongest desorption strength of the eluent passing through the section 1, weakening desorption strength of the eluent passing through the section 2 more than the desorption strength of the eluent passing through the section 1, weakening desorption strength of the eluent passing through the section 3 more than the desorption strength of the eluent passing through the section 2, and weakening desorption strength of the eluent passing through the section 4 more than the desorption strength of the eluent passing through the section 3; and
<sub-step (A4-1)>
supplying an eluent d-II from an eluent supply port D-II while using an upstream end of the section 2 as the eluent supply port D-II, supplying an eluent d-III from an eluent supply port D-III while using an upstream end of the section 3 as the eluent supply port D-III, supplying an eluent d-IV from an eluent supply port D-IV while using an upstream end of the section 4 as the eluent supply port D-IV, and extracting the weakly adsorptive fraction from a weakly adsorptive fraction extraction port A while using a downstream end of the section 1 as the extraction port A,
thereby most strengthening strongest desorption strength of the eluent passing through the section 2, weakening desorption strength of the eluent passing through the section 3 more than the desorption strength of the eluent passing through the section 2, weakening desorption strength of the eluent passing through the section 4 more than the desorption strength of the eluent passing through the section 3, and making the same desorption strength of the eluent passing through the section 1 as the desorption strength of the eluent passing through the section 4;
<sub-step (B1-1)>
supplying an eluent d-II from an eluent supply port D-II while using an upstream end of the section 2 as the eluent supply port D-II, supplying an eluent d-III from an eluent supply port D-III while using an upstream end of the section 4 as the eluent supply port D-III, supplying an eluent d-IV from an eluent supply port D-IV while using an upstream end of the section 1 as the eluent supply port D-IV, and extracting the weakly adsorptive fraction from a weakly adsorptive fraction extraction port A while using a downstream end of the section 1 as the extraction port A,
thereby most strengthening strongest desorption strength of the eluent passing through the sections 2 and 3, weakening desorption strength of the eluent passing through the section 4 more than the desorption strength of the eluent passing through the sections 2 and 3, and weakening desorption strength of the eluent passing through the section 1 more than the desorption strength of the eluent passing through the section 4;
<sub-step (B2-1)>
supplying an eluent d-I from an eluent supply port D-I while using an upstream end of the section 2 as the eluent supply port D-I, extracting the strongly adsorptive fraction from a strongly adsorptive fraction extraction port C while using a downstream end of the section 2 as the extraction port C, supplying an eluent d-II from an eluent supply port D-II while using an upstream end of the section 3 as the eluent supply port D-II, supplying an eluent d-III from an eluent supply port D-III while using an upstream end of the section 4 as the eluent supply port D-III, supplying an eluent d-IV from an eluent supply port D-IV while using an upstream end of the section 1 as the eluent supply port D-IV, and extracting the weakly adsorptive fraction from a weakly adsorptive fraction extraction port A while using a downstream end of the section 1 as the extraction port A,
thereby most strengthening strongest desorption strength of the eluent passing through the section 2, weakening desorption strength of the eluent passing through the section 3 more than the desorption strength of the eluent passing through the section 2, weakening desorption strength of the eluent passing through the section 4 more than the desorption strength of the eluent passing through the section 3, and weakening desorption strength of the eluent passing through the section 1 more than the desorption strength of the eluent passing through the section 4;
<sub-step (B3-1)>
supplying an eluent d-II from an eluent supply port D-II while using an upstream end of the section 3 as the eluent supply port D-II, supplying an eluent d-III from an eluent supply port D-III while using an upstream end of the section 4 as the eluent supply port D-III, supplying an eluent d-IV from an eluent supply port D-IV while using an upstream end of the section 1 as the eluent supply port D-IV, and extracting the weakly adsorptive fraction from a weakly adsorptive fraction extraction port A while using a downstream end of the section 2 as the extraction port A,
thereby most strengthening strongest desorption strength of the eluent passing through the section 3, weakening desorption strength of the eluent passing through the section 4 more than the desorption strength of the eluent passing through the section 3, weakening desorption strength of the eluent passing through the section 1 more than the desorption strength of the eluent passing through the section 4, and making the same desorption strength of the eluent passing through the section 2 as the desorption strength of the eluent passing through the section 1;
<sub-step (B1'-1)>
supplying an eluent d-II from an eluent supply port D-II while using an upstream end of the section 3 as the eluent supply port D-II, supplying an eluent d-III from an eluent supply port D-III while using an upstream end of the section 1 as the eluent supply port D-III, supplying an eluent d-IV from an eluent supply port D-IV while using an upstream end of the section 2 as the eluent supply port D-IV, and extracting the weakly adsorptive fraction from a weakly adsorptive fraction extraction port A while using a downstream end of the section 2 as the extraction port A,
thereby most strengthening strongest desorption strength of the eluent passing through the sections 3 and 4, weakening desorption strength of the eluent passing through the section 1 more than the desorption strength of the eluent passing through the sections 3 and 4, and weakening desorption strength of the eluent passing through the section 2 more than the desorption strength of the eluent passing through the section 1;
<sub-step (B2'-1)>
supplying an eluent d-I from an eluent supply port D-I while using an upstream end of the section 3 as the eluent supply port D-I, extracting the strongly adsorptive fraction from a strongly adsorptive fraction extraction port C while using a downstream end of the section 3 as the extraction port C, supplying an eluent d-II from an eluent supply port D-II while using an upstream end of the section 4 as the eluent supply port D-II, supplying an eluent d-III from an eluent supply port D-III while using an upstream end of the section 1 as the eluent supply port D-III, supplying an eluent d-IV from an eluent supply port D-IV while using an upstream end of the section 2 as the eluent supply port D-IV, and extracting the weakly adsorptive fraction from a weakly adsorptive fraction extraction port A while using a downstream end of the section 2 as the extraction port A,
thereby most strengthening strongest desorption strength of the eluent passing through the section 3, weakening desorption strength of the eluent passing through the section 4 more than the desorption strength of the eluent passing through the section 3, weakening desorption strength of the eluent passing through the section 1 more than the desorption strength of the eluent passing through the section 4, and weakening desorption strength of the eluent passing through the section 2 more than the desorption strength of the eluent passing through the section 1; and
<sub-step (B3'-1)>
supplying an eluent d-II from an eluent supply port D-II while using an upstream end of the section 4 as the eluent supply port D-II, supplying an eluent d-III from an eluent supply port D-III while using an upstream end of the section 1 as the eluent supply port D-III, supplying an eluent d-IV from an eluent supply port D-IV while using an upstream end of the section 2 as the eluent supply port D-IV, and extracting the weakly adsorptive fraction from a weakly adsorptive fraction extraction port A while using a downstream end of the section 3 as the extraction port A,
thereby most strengthening strongest desorption strength of the eluent passing through the section 4, weakening desorption strength of the eluent passing through the section 1 more than the desorption strength of the eluent passing through the section 4, weakening desorption strength of the eluent passing through the section 2 more than the desorption strength of the eluent passing through the section 1, and making the same desorption strength of the eluent passing through the section 3 as the desorption strength of the eluent passing through the section 2.

6. The simulated moving-bed type chromatographic separation method according to any one of claims 2 to 4, comprising: using a circulation system in which three or more unit packed columns each filled with an adsorbent are connected in series and in an endless form via pipes, and dividing the circulation system into three sections annularly continuous from an upstream side to a downstream side so that each section has at least one of the unit packed column, wherein sub-steps (A1) to (A4) in step (A) are sub-steps (A1-2) to (A4-2) below, and
wherein, in step (B), the following sub-steps (B1-2) and (B2-2) are performed in sequence:
<sub-step (A1-2)>
supplying an eluent d-II from an eluent supply port D-II while using an upstream end of the section 1 as the eluent supply port D-II, extracting the intermediately adsorptive fraction from an intermediately adsorptive fraction extraction port B while using a downstream end of the section 2 as the extraction port B, supplying the feed solution from a feed solution supply port F while using an upstream end of the section 3 as the feed solution supply port F, and extracting the weakly adsorptive fraction from a weakly adsorptive fraction extraction port A while using a downstream end of the section 3 as the extraction port A,
thereby most strengthening strongest desorption strength of the eluent passing through the sections 1 and 2, and weakening desorption strength of the eluent passing through the section 3 more than the desorption strength of the eluent passing through the sections 1 and 2;
<sub-step (A2-2)>
supplying an eluent d-II from an eluent supply port D-II while using an upstream end of the section 1 as the eluent supply port D-II, extracting the intermediately adsorptive fraction from an intermediately adsorptive fraction extraction port B while using a downstream end of the section 2 as the extraction port B, supplying an eluent d-IV from an eluent supply port D-IV while using an upstream end of the section 3 as the eluent supply port D-IV, and extracting the weakly adsorptive fraction from a weakly adsorptive fraction extraction port A while using a downstream end of the section 3 as the extraction port A,
thereby most strengthening strongest desorption strength of the eluent passing through the sections 1 and 2, and weakening desorption strength of the eluent passing through the section 3 more than the desorption strength of the eluent passing through the sections 1 and 2;
<sub-step (A3-2)>
supplying an eluent d-I from an eluent supply port D-I while using an upstream end of the section 1 as the eluent supply port D-I, extracting the strongly adsorptive fraction from a strongly adsorptive fraction extraction port C while using a downstream end of the section 1 as the extraction port C, supplying an eluent d-II from an eluent supply port D-II while using an upstream end of the section 2 as the eluent supply port D-II, supplying an eluent d-III from an eluent supply port D-III while using an upstream end of the section 3 as the eluent supply port D-III, and extracting the weakly adsorptive fraction from a weakly adsorptive fraction extraction port A while using a downstream end of the section 3 as the extraction port A,
thereby most strengthening strongest desorption strength of the eluent passing through the section 1, weakening desorption strength of the eluent passing through the section 2 more than the desorption strength of the eluent passing through the section 1, and weakening desorption strength of the eluent passing through the section 3 more than the desorption strength of the eluent passing through the section 2;
<sub-step (A4-2)>
supplying an eluent d-II from an eluent supply port D-II while using an upstream end of the section 2 as the eluent supply port D-II, supplying an eluent d-III from an eluent supply port D-III while using an upstream end of the section 3 as the eluent supply port D-III, supplying an eluent d-IV from an eluent supply port D-IV while using an upstream end of the section 1 as the eluent supply port D-IV, and extracting the weakly adsorptive fraction from a weakly adsorptive fraction extraction port A while using a downstream end of the section 1 as the extraction port A,
thereby most strengthening strongest desorption strength of the eluent passing through the section 2, weakening desorption strength of the eluent passing through the section 3 more than the desorption strength of the eluent passing through the section 2, and weakening desorption strength of the eluent passing through the section 1 more than the desorption strength of the eluent passing through the section 3;
<sub-step (B1-2)>
supplying an eluent d-I from an eluent supply port D-I while using an upstream end of the section 2 as the eluent supply port D-I, extracting the strongly adsorptive fraction from a strongly adsorptive fraction extraction port C while using a downstream end of the section 2 as the extraction port C, supplying an eluent d-II from an eluent supply port D-II while using an upstream end of the section 3 as the eluent supply port D-II, supplying an eluent d-III from an eluent supply port D-III while using an upstream end of the section 1 as the eluent supply port D-III, and extracting the weakly adsorptive fraction from a weakly adsorptive fraction extraction port A while using a downstream end of the section 1 as the extraction port A,
thereby most strengthening strongest desorption strength of the eluent passing through the section 2, weakening desorption strength of the eluent passing through the section 3 more than the desorption strength of the eluent passing through the section 2, and weakening desorption strength of the eluent passing through the section 1 more than the desorption strength of the eluent passing through the section 3; and
<sub-step (B2-2)>
supplying an eluent d-II from an eluent supply port D-II while using an upstream end of the section 3 as the eluent supply port D-II, supplying an eluent d-III from an eluent supply port D-III while using an upstream end of the section 1 as the eluent supply port D-III, supplying an eluent d-IV from an eluent supply port D-IV while using an upstream end of the section 2 as the eluent supply port D-IV, and extracting the weakly adsorptive fraction from a weakly adsorptive fraction extraction port A while using a downstream end of the section 2 as the extraction port A,
thereby most strengthening strongest desorption strength of the eluent passing through the section 3, weakening desorption strength of the eluent passing through the section 1 more than the desorption strength of the eluent passing through the section 3, and weakening desorption strength of the eluent passing through the section 2 more than the desorption strength of the eluent passing through the section 1.

7. The simulated moving-bed type chromatographic separation method according to any one of claims 1 to 6, wherein the intermediately adsorptive component is a biopolymer.

8. A simulated moving-bed type chromatographic separation system obtained by using a circulation system in which three or more unit packed columns each filled with an adsorbent are connected in series and in an endless form via pipes, dividing the circulation system into at least three sections annularly continuous from an upstream side to a downstream side so that each section has at least one of the unit packed column, and separating, by using an eluent, a weakly adsorptive component, a strongly adsorptive component, and an intermediately adsorptive component having adsorption performance on the adsorbent, which performance is intermediate between the previous two components, the weakly adsorptive component, strongly adsorptive component, and intermediately adsorptive component being included in a feed solution, the system comprising means for repeating a cycle containing a first process and a second process below:
<first process>
a process containing blocking circulation of the circulation system by setting a blocked position to a position upstream of the weakly adsorptive component-rich section, supplying the feed solution to a section downstream of the blocked position, and extracting, from the circulation system, the intermediately adsorptive component-rich intermediately adsorptive fraction from a section upstream of the blocked position; and
<second process>
a process containing extracting the strongly adsorptive component-rich strongly adsorptive fraction by controlling supply of the eluent and blocking of the circulation system without supplying the feed solution, and shifting a supply position of the eluent relative to the circulation system and an extraction position of the strongly adsorptive fraction to a downstream side in accordance with movement of the strongly adsorptive fraction, and
wherein in the cycle containing the first process and the second process, the extraction position of the strongly adsorptive fraction is shifted to a downstream side by 2 sections or more and the [number of sections] shifted toward the downstream side and the total number of sections are not the same.

9. A simulated moving-bed type chromatographic separation system obtained by using a circulation system in which three or more unit packed columns each filled with an adsorbent are connected in series and in an endless form via pipes, dividing the circulation system into at least three sections annularly continuous from an upstream side to a downstream side so that each section has at least one of the unit packed column, and separating, by using an eluent, a weakly adsorptive component, a strongly adsorptive component, and an intermediately adsorptive component having adsorption performance on the adsorbent, which performance is intermediate between the previous two components, the weakly adsorptive component, strongly adsorptive component, and intermediately adsorptive component being included in a feed solution, the system comprising means for repeating a cycle containing step (A) and step (B) below:
<step (A)>
a step comprising:
sub-step (A1) of blocking circulation of the circulation system by setting a blocked position to a position upstream of the weakly adsorptive component-rich section, supplying the feed solution at a section downstream of the blocked position and extracting, from the circulation system, the weakly adsorptive component-rich weakly adsorptive fraction at a position further downstream of the blocked position, and supplying an eluent for desorbing the intermediately adsorptive component at a section upstream of the blocked position and extracting, from the circulation system, the intermediately adsorptive component-rich intermediately adsorptive fraction at a position downstream thereof,
sub-step (A2) of supplying an eluent for desorbing the weakly adsorptive component instead of supplying the feed solution and continuing extraction of the weakly adsorptive fraction and extraction of the intermediately adsorptive fraction,
sub-step (A3) of shifting the blocked position to an upstream side and supplying an eluent for desorbing the strongly adsorptive component at a position upstream of the blocked position having been shifted and extracting the strongly adsorptive component-rich strongly adsorptive fraction at a position further downstream thereof while continuing extraction of the weakly adsorptive fraction, and
sub-step (A4) of shifting the extraction position of the weakly adsorptive fraction to a downstream side and extracting the weakly adsorptive fraction while extracting neither the intermediately adsorptive fraction nor the strongly adsorptive fraction,
wherein, in step (A), the sub-steps (A1), (A2), (A3) and (A4) are performed in this sequence; and
<step (B)>
a step comprising step α of separately extracting each of the weakly adsorptive fraction or strongly adsorptive fraction not extracted in step (A) by controlling supply of the eluent and the blocked position without supplying the feed solution while keeping the extraction position of the weakly adsorptive fraction as it is, and then shifting the extraction position of the weakly adsorptive fraction to a downstream side and extracting only the weakly adsorptive fraction, wherein, in step (B), at least one of the step α is performed.
